# EUROPEAN PATENT SPECIFICATION

(11) **EP 2 184 077 B1**
(45) Date of publication and mention of the grant of the patent: **14.05.2014**
(21) Application number: 09013159.0
(22) Date of filing: 06.12.2005
(51) Int. Cl.: A61L 24/10

(54) **Materials and methods for minimally-invasive administration of a cell-containing flowable composition**
Materialien und Verfahren zur minimal invasiven Abgabe einer zellhaltigen fließfähigen Zusammensetzung
Matériaux et procédés d'administration invasive minimale d'une composition fluide contenant une cellule

(30) Priority: 08.12.2004 US 634155 P; 21.03.2005 US 663859 P; 18.05.2005 US 682054 P
(43) Date of publication of application: 12.05.2010
(62) Divisional of application: 05848586.3
(73) Proprietor: Shire Regenerative Medicine, Inc., San Diego, California 92121 (US)
(72) Inventor: Nugent, Helen Marie, Needham, MA 02492 (US); Edelman, Elazer, Brookline, MA 02146 (US); Bollinger, Steve, Mansfield, MA 02048 (US)
(74) Representative: Lock, Graham James

(56) References cited:
- US-A1- 2001 036 451
- US-A1- 2002 090 398
- US-A1- 2003 163 192
- US-B2- 6 723 131

## Description

### Background of the Invention

Cardiovascular disease accounts for over 50 million deaths in the world and 1 million deaths in the United States each year. Approximately 1.5 million procedures are performed each year in the United States in an attempt to alleviate the obstructive arterial lesions that spontaneously arise from these diseases. These procedures include balloon and laser angioplasty, atherectomy, endovascular stents and bypass grafts, to name but a few. The long-term effectiveness of angioplasty, vascular bypass grafts and even organ transplantation is limited by accelerated arteriopathies following such procedures. The loss of endothelial integrity, occlusive thrombosis, spasm and the migration and proliferation of smooth muscle cells resulting in intimal hyperplasia typify such arteriopathies. For example, restenosis leads to obstructive arterial lesions in 20 to 50% of such patients. Within three to six months after coronary angioplasty, over a third of patients require additional intervention, another angioplasty or even bypass surgery. Atherectomy devices are no better; as the number of patients subjected to this procedure increases, the rates of restenosis have climbed from 10 to 47%. The use of endovascular stents has also been somewhat disappointing in this regard. A recent study reports a 35% rate of restenosis in addition to the approximately 5% of patients who suffer an acute complication, such as abrupt closure within the first few days after insertion.

Similar problems have been observed in patients who undergo vascular bypass surgery. The mean lifetime of a saphenous venous aorto-coronary interposition graft is only seven years. Ten percent of all such grafts are occluded within the first weeks after surgery. At one and five years, 20 and 35% of grafts are occluded, respectively. Arterio-venous fistulae in dialysis patients are subject to the same pathology limiting the efficacy of hemodialysis.

The hallmark of accelerated arteriopathies, such as restenosis, is exuberant smooth muscle cell proliferation and the deposition of a large amount of extracellular matrix generated by these cells. It has now become evident that both native atherosclerosis and the accelerated arteriopathies that follow mechanical interventions share a common initial pathological event, loss of endothelial cell integrity and function.

The endothelial monolayer lines the arterial wall and serves as a two-fold bioregulator of vascular physiology. The endothelium provides structural integrity to the blood vessel by forming a continuous, selectively permeable, nonthrombogenic barrier between circulating blood and the arterial wall. Yet, it is becoming increasingly appreciated that the endothelium also produces and supplies products which control blood flow, vessel tone, occlusive thrombosis, platelet activation, adhesion, and aggregation, leukocyte adhesion, monocyte infiltration and smooth muscle cell migration and proliferation. Since smooth muscle cell mitogens are ubiquitous within the arterial wall, it is the presence of an intact endothelium that maintains the normal blood vessel in a quiescent state. Upon damage or removal of the endothelium, compounds secreted by the endothelium are removed as well, and a sequence of events is set into motion that leads to the uncontrolled proliferation and migration of smooth muscle cells resulting in obstructive arterial lesions.

Many clinical interventions currently employed to treat cardiovascular diseases, including coronary angioplasty, coronary stenting and atherectomy, can be accomplished using non-invasive closed surgical procedures. These non-invasive endovascular intervention strategies should be accompanied by a similarly minimally-invasive endovascular mode of delivering a therapeutic material to the site of vascular intervention to treat the resulting injured or diseased target endothelium.

Furthermore, current methods of delivering therapeutic materials endovascularly rely on administration of the materials to the interior luminal surface of the blood vessel. However, administration of therapeutic materials or agents to the interior luminal surface provides only transient benefit to an injured or diseased target endothelium as contact with circulating blood limits efficacy and duration of activity.

US-A-8 723 131, concerns a composite bone marrow graft material comprising a porous biocompatible implantable matrix being enriched with a population of progenilor cells and a clot material in form of a blood clot, bone marrow, platelet gel or a fibrin clot material, said matrix composed of a porous biocompatible surface.

US-A-2003/0163192, refers to a method for treating a diseased artery comprising the delivery of a fluidic composition to the affected vascular site under conditions where the the composition in-situ forms a solid film adhering to the vascular wall, thus isolating the vascular site from systemic blood flow.

US-A-2002/0090398, discloses a flowable composition containing a matrix of a biocompatible polymer and a bioactive agent, whereby said composition can be administered within a solid matrix in-situ as an implant.

US-A-2001/0036451, refers to embolic compositions comprising a polymeric backbone containing chains boaring crosslinkable groups capable of forming hydrogels by crosslinking.

WO 2004/084921 discloses perivascular mesenchymal progenitor cells for use in inducing new blood vessel formation.

### Summary of Invention

The present invention exploits the discovery that cells engrafted in, on or within an implantable flowable composition can be formulated for multiple modes of minimally-invasive delivery, for example, endovascular administration and perivascular deposition at, adjacent or in the vicinity of an extraluminal surface of a tubular anatomical structure such as but not limited to a blood vessel. Minimally-invasive delivery at, on or around an exterior surface of a tubular anatomical structure is also contemplated herein. In the case of blood vessel, the materials of the present invention are suitable for treating and managing clinical sequelae associated with vascular interventions or cardiovascular diseases.

In one aspect, the present invention provides a flowable composition as defined in the claims.

According to one embodiment, the tubular anatomical structure is a blood vessel. The flowable composition is provided, according to some embodiments, in an amount effective to reduce smooth muscle cell proliferation, occlusive thrombosis, intimal hyperplasia, restenosis, acute or chronic inflammation or vasodilation, to name but a few, at the injured or diseased site. For purposes of the present invention, flowable composition means a composition susceptible to administration using an injection or injection-type delivery device such as, but not limited to, a needle, a syringe or a catheter. Other delivery devices which employ extrusion, ejection or expulsion are also contemplated herein.

According to one embodiment, the cells of the flowable composition are endothelial cells or cells having an endothelial-like phenotype. According to another embodiment, the cells are a co-culture of two or more cell types. The two or more cell types are selected from the group consisting of endothelial cells, epithelial cells, smooth muscle cells, fibroblasts, stem cells, endothelial progenitor cells and cardiomyocytes. A preparation of cells suitable for use with the present invention can be obtained from a single donor or multiple donors.

According to another embodiment, the biocompatible matrix is a gel, a foam, or a suspension. The biocompatible matrix, in yet another embodiment, comprises particles or microcarriers. In certain embodiments, the particles or microcarriers further comprise gelatin, collagen, fibronectin, fibrin, laminin or attachment peptide. One exemplary attachment peptide is a peptide of sequence arginine-glycine-aspartate (RGD). According to another embodiment, the particle or microcarrier has a diameter of about 20 microns to about 500 microns, preferably a diameter of about 200 microns.

In another embodiment, the flowable composition further comprises a carrier fluid. In a particularly preferred embodiment, the flowable composition is shape-retaining, thereby permitting the practitioner to control deposition to an extent necessary given a particular deposition site.

The present invention finds application in a method of treating an injured or diseased site on an interior lumen of a tubular anatomical structure comprising the step of contacting with a flowable composition an extraluminal surface of the tubular anatomical structure at or adjacent or in the vicinity of the injured or diseased site on the interior lumen of the tubular anatomical structure. It is contemplated herein that a non-luminal, also termed an extraluminal, surface can be an exterior or perivascular surface of a vessel., For purposes of this invention, non-luminal or extraluminal site is any site except an interior surface of the lumen. In the case of a blood vessel, for example, an extraluminal or non-luminal site can be within the adventitia, media, or intima of a blood vessel; in the case of non-vascular tubular anatomical structures, corresponding non-luminal sites are within the scope of the present invention.

According to one embodiment, delivery is accomplished by traversing or penetrating an interior wall of the tubular anatomical structure and then depositing the flowable composition on an exterior surface of the tubular anatomical structure at or adjacent or in the vicinity of the injured or diseased site. According to another embodiment, the method further comprises the step of identifying a site for depositing the flowable composition on an extraluminal surface of the tubular anatomical structure. According to one embodiment, the identifying step occurs prior to or coincident with the traversing or penetrating step. The identifying step, in one embodiment, is accomplished by imaging. The identifying step, in another embodiment, is accomplished by tactile palpation.

In one embodiment, delivery is accomplished by entering the perivascular space by percutaneous administration and then depositing the flowable composition on an exterior surface of the tubular anatomical structure at or adjacent or in the vicinity of the injured or diseased state. According to another embodiment, this method further comprises the step of identifying a site for depositing the flowable composition on an exterior surface of the tubular anatomical structure. The identifying step can occur prior to or coincident with the entering step. The identifying step, according to one embodiment, is accomplished by imaging. The identifying step, in another embodiment, is accomplished by tactile palpation.

The exterior surface of the tubular anatomical structure may be a non-luminal surface or may occupy a perivascular space as described elsewhere herein. According to one preferred embodiment, the tubular anatomical structure is a blood vessel. According to another preferred embodiment, the blood vessel comprises a stent. In yet anther preferred embodiment, the treated tubular anatomical structure is a non-vascular structure such as, but not limited to, a fallopian tube.

### Brief Description of the Drawings

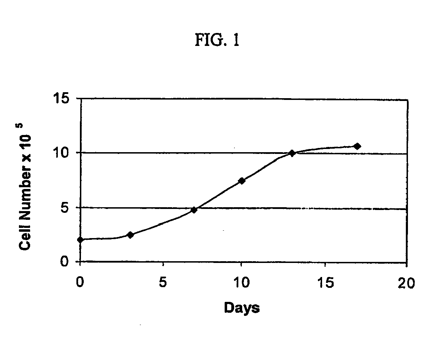

FIG. 1 is a representative cell growth curve according to an illustrative embodiment of the invention.

### Detailed Description of the Invention

As explained herein, the invention is based on the discovery that a cell-based therapy can be used to treat, ameliorate, manage and/or reduce the progression of clinical sequelae associated with vascular interventions or cardiovascular diseases, particularly occlusive thrombosis, restenosis, intimal hyperplasia, inflammation and vasodilation. The invention further benefits from the additional discovery that a heretofore undescribed flowable composition, for example a particulate formulation, is capable of sustaining a confluent population of sufficiently viable cells and that this composition comprising cells engrafted in, on or within a biocompatible matrix, for example an implantable particulate material, can be effectively administered using a minimally-invasive mode of administration, for example an endovascular or local percutaneous delivery during a closed procedure, without diminishing the clinical effectiveness or the viability of the implantable flowable composition's engrafted cells. The teachings presented below provide sufficient guidance to make and use the materials of the present invention, and further provide sufficient guidance to identify suitable criteria and subjects for testing, measuring, and monitoring the performance of the materials of the present invention.

Accordingly, the present invention finds application in a cell-based therapy for clinically managing vascular interventions or cardiovascular diseases. An exemplary embodiment of the present invention comprises a biocompatible matrix and cells suitable for use with the treatment paradigms described herein. Specifically, in one preferred embodiment, the implantable flowable composition comprises a biocompatible matrix and endothelial cells or endothelial-like cells. In another preferred embodiment, the implantable flowable composition comprises endothelial cells or endothelial-like cells, preferably human aortic endothelial cells and a particulate-type biocompatible matrix.

Implantable flowable composition of the present invention comprises cells engrafted on, in and/or within a biocompatible matrix. Engrafted means securely attached via cell to cell and/or cell to matrix interactions such that the cells withstand the rigors of the preparatory manipulations disclosed herein. As explained elsewhere herein, an operative embodiment of implantable flowable composition comprises a near-confluent, confluent or post-confluent cell population having a preferred phenotype. It is understood that embodiments of implantable flowable composition likely shed cells during preparatory manipulations and/or that certain cells are not as securedly attached as are other cells. All that is required is that implantable flowable composition comprise cells that meet the functional or phenotypical criteria set forth herein.

Implantable flowable composition of the present invention was developed on the principles of tissue engineering and represents a novel approach to addressing the above-described clinical needs. The implantable flowable composition of the present invention is unique in that the viable cells engrafted on, in and/or within the biocompatible matrix are able to supply to the tubular anatomical structure multiple cell-based products in physiological proportions under physiological fee-back control. As described elsewhere herein, cells suitable for use with the implantable flowable composition are endothelial or endothelial-like cells. Local delivery of multiple compounds by these cells and a physiologically-dynamic dosing provide more effective regulation of the processes responsible for maintaining a functional luminal endothelium. Importantly, the endothelial cells, for example, in the implantable flowable composition of the present invention are protected from the erosive blood flow within a blood vessel's interior lumen because of its preferred placement at a non-luminal or extraluminal site.

The implantable flowable composition of the present invention, when wrapped, deposited or otherwise contacted with an extraluminal or non-luminal site at, adjacent or in the vicinity of an injured or diseased target lumen, serves to reestablish homeostatis. That is, the implantable flowable composition of the present invention when administered extraluminally can provide an environment which mimics supportive physiology and is conducive to promoting functional interior lumen. As contemplated herein, tubular anatomical structures are those having an interior luminal surface and an extraluminal surface. In certain structures, the interior luminal surface is an endothelial cell layer; in certain other structures, the interior luminal surface is a non-endothelial cell layer. Again, for purposes of the present invention, an extraluminal or non-luminal surface can be but is not limited to an exterior surface of a tubular structure as described elsewhere herein.

For example, endothelial cells can release a wide variety of agents that in combination can inhibit or mitigate adverse physiological events associated with acute complications following vascular intervention or cardiovascular disease. As exemplified herein, a composition and method of use that recapitulates normal physiology and dosing is useful to enhance endothelium functionality as well as promote long-term patency of such lumen endothelium. Typically, treatment includes depositing the implantable flowable composition of the present invention at, adjacent or in the vicinity of an injured or diseased target endothelium, for example, in the perivascular space external to the lumen of the subject vasculature. When deposited or otherwise contacting an injured, traumatized or diseased blood vessel, the cells of the implantable flowable composition can provide growth regulatory compounds to the subject vasculature, for example to the underlying smooth muscle cells within the blood vessel. While outside the blood vessel, the implantable flowable composition of the present invention provides an effective supply of multiple regulatory compounds from the cells while being protected from the mechanical effects of blood flow in the interior lumen of the vessel(s).

Treatment of an injured or diseased blood vessel with a preferred embodiment of the present invention can encourage normal or near-normal healing and normal physiology. In contrast, in the absence of treatment with a preferred embodiment of the present invention, normal physiological healing is impaired, e.g., native endothelial cells and smooth muscle cells can grow abnormally at an exuberant or uncontrolled rate following vascular intervention or cardiovascular disease. Accordingly, as contemplated herein, treatment with the implantable flowable composition of the present invention will result in normal or near-normal healing of native tissue at the site of vascular intervention or cardiovascular disease, for example, sufficient to maintain normal or near-normal vessel patency.

The implantable flowable composition of the present invention can be placed in a variety of configurations at the vasculature to be treated. The vessels can be contacted in whole or in part; for example, the implantable flowable composition of the present invention can be applied to the vessels circumferentially or in an arc configuration. A vessel need only be in contact with an amount of implantable flowable composition sufficient to improve functionality of the vasculature.

For purposes of the present invention, contacting means directly or indirectly interacting with an extraluminal or non-luminal surface as defined elsewhere herein. In the case of certain preferred embodiments, actual physical contact is not required for effectiveness. In other embodiments, actual physical contact is preferred. All that is required to practice the present invention is extraluminal or non-luminal deposition of an implantable material at, adjacent or in the vicinity of an injured or diseased site in an amount effective to treat the injured or diseased site. In the case of certain diseases or injuries, a diseased or injured site can clinically manifest on an interior lumen surface. In the case of other diseases or injuries, a diseased or injured site can clinically manifest on an extraluminal or non-luminal surface. In some diseases or injuries, a diseased or injured site can clinically manifest on both an interior lumen surface and an extraluminal or non-luminal surface. The present invention is effective to treat any of the foregoing clinical manifestations.

Embodiments of the implantable flowable composition of the present invention can be applied to any tubular anatomical structure requiring interventional therapy to maintain homeostasis. As contemplated herein, tubular anatomical structures are those having an interior luminal surface and an extraluminal or non-luminal surface. For purposes of the present invention, an extraluminal surface can be, but is not limited to, an exterior surface of a tubular structure. In certain tubular structures, the interior luminal surface is an endothelial cell layer; in certain other structures, the interior luminal surface is a non-endothelial cell layer. The present invention is effective to treat an endothelial-lined or non-endothelial-lined tubular structure.

Tubular anatomical structures include structures of the vascular system, the reproductive system, the genitourinary system, the gastrointestinal system, the pulmonary system, the respiratory system and the ventricular system of the brain and spinal cord. Representative examples of tubular anatomical structures include arteries and veins, lacrimal ducts, the trachea, bronchi, bronchiole, nasal passages (including the sinuses) and other airways, eustachian tubes, the external auditory canal, oral cavities, the esophagus, the stomach, the duodenum, the small intestine, the large intestine, biliary tracts, the ureter, the bladder, the urethra, the fallopian tubes, uterus, vagina and other passageways of the female reproductive tract, the vasdeferens and other passages of the male reproductive tract, a vascular sheath, and the ventricular system (cerebrospinal fluid) of the brain and spinal cord. For purposes of the present invention, tubular anatomical structures can be naturally-occurring or non-naturally occurring such as but not limited to a surgically created anastomoses.

Injured or Diseased Endothelium: In certain preferred embodiments, vascular interventions resulting in vascular injuries susceptible to treatment with the present invention include but are not limited to angioplasty, atherectomy, vascular stenting including bare-metal and drug-eluting stents, vascular bypass surgeries including arterial bypass grafts and peripheral bypass grafts, organ transplantation, arteriovenous fistula and other vascular anastomosis formation, arteriovenous, peripheral and other graft formation, and subsequent vascular access-associated injuries, including needle sticks incurred during accessing a vessel for dialysis or other interventional therapy. Each intervention results in a degree of injury to the endothelial cell lining of the vascular lumen. In turn, the injured vascular lumen experiences a cascade of biochemical events resulting in a variety of clinically identifiable sequelae, including but not limited to occlusive thrombosis, restenosis, intimal hyperplasia, acute and chronic inflammation, smooth muscle cell proliferation, vascular remodeling, vasodilation and the formation of vulnerable plaque lesions.

Thrombosis or occlusive thrombosis is associated with platelet adhesion, aggregation and organization; occlusive thrombosis is generally associated with an organized thrombus. Thrombosis is characterized by loss of blood flow in the thrombosed area. Endothelial or endothelial-like cells release anti-thrombotic compounds including but not limited to heparan sulfate proteoglycans, prostacyclin and nitric oxide. Treatment with the implantable flowable composition of the present invention improves the patency of the treated vessel.

Stenosis, restenosis, intimal hyperplasia and smooth muscle cell proliferation are characterized by obstructive lesions in the lumen of a blood vessel associated with exhuberant smooth muscle cell growth into the lumen. Endothelial or endothelial-like cells release compounds into the lumenal area that inhibit smooth muscle cell proliferation. Exemplary therapeutic compounds produced by endothelial or endothelial-like cells include but are not limited to heparan sulfate, TGF-β and nitric oxide. Stenosis, restenosis, intimal hyperplasia and smooth muscle cell proliferation are identified, for example, by angiography, intravascular ultrasound or other ultrasound techniques. Treatment with the implantable flowable composition of the present invention diminishes the percent stenosis, extent of occlusion and/or improves patency associated with the treated vessel.

Inflammation is associated with recruitment, adhesion and infiltration of inflammatory cells, including but not limited to granulocytes, neutrophils, monocytes, macrophages and lymphocytes. In addition, an increase in vascular permeability leads to a local accumulation of fluid, immunoglobulins, complement, and other blood proteins in the tissue adjacent a site of injury which, in turn, induce the expression of adhesion molecules, which bind to the surface of circulating monocytes and neutrophils and greatly enhance the rate at which these phagocytic cells can migrate across the lumen surface and into the adjacent tissue. Upon activation, these cells can release hydrolytic enzymes, cytokines, chemokines and growth factors. In advanced, chronic stages of inflammation, the injured site becomes covered by a fibrous cap that overlies a core of lipid and necrotic tissue. Endothelial or endothelial-like cells release anti-inflammatory compounds into the lumenal area that reduce the inflammatory response. Treatment with the implantable flowable composition of the present invention can inhibit the activity and/or accumulation of inflammatory cells, thereby decreasing production and secretion of growth factors and decreasing local vascular infiltration of macrophages to prevent, reduce or ameliorate the acute inflammatory response at the site of vascular injury. Amelioration or prevention of the acute inflammatory response can interrupt events leading to chronic inflammation, thereby minimizing eventual luminal compromise and/or vascular dysfunction. Additionally, amelioration or rehabilitation of chronically inflamed tissue can reduce the risks of onset of long-term risks such as vascular diseases including but not limited to vulnerable plaque or atherosclerosis.

Moreover, spontaneously occurring cardiovascular diseases which are susceptible to treatment with the present invention include but are not limited to acute and chronic inflammation, occlusive thrombosis, intimal hyperplasia, restenosis, smooth muscle cell proliferation, vasodilation, negative vascular remodeling, vulnerable plaque lesions within the lumen of the vascular structure, and various unstable arterial syndromes to name but a few. Additional vulnerable vascular conditions which are susceptible to treatment with the present invention include any ischemic, hypoxic or injured state where there is an inadequate blood supply relative to demand. Vulnerable vascular conditions can result from any injury or repair that negatively impact blood supply. Exemplary vulnerabilities include unstable arterial syndromes such as ischemia, unstable angina in the heart including a spectrum of instability ranging from exercise induced angina to angina at rest, aortic ischemia, peripheral ischemias including a spectrum of conditions ranging from intermittent caludication to gangrene, bowel ischemia in the gut and renal ischemia, to name but a few.

An injured or diseased target endothelium may be treated with the implantable flowable composition of the present invention at the time of a primary vascular intervention, for example, angioplasty, stenting or anastomosis creation. Such treatment can diminish injury resulting from the vascular intervention, for example, endothelial denuding resulting from angioplasty. The implantable flowable composition may also be administered to rescue an injured or diseased target endothelium subsequent to a vascular intervention and development of intervention-associated clinical arteriopathies, including but not limited to, for example, restenosis or occlusive thrombosis.

Additional therapeutic agents may be administered prior to, coincident with and/or following administration of the implantable flowable composition. For example, agents which prevent or diminish blood clot formation, platelet aggregation or other similar blockages can be administered. Exemplary agents include, for example, heparan sulfate and TGF-β. Other cytokines or growth factors can also be incorporated into the implantable flowable composition, depending on the indication of the implant, including VEGF to promote reendothelialization and b-FGF to promote vessel integration. Other types of therapeutic agents include, but are not limited to, antiproliferative agents and antineoplastic agents. Examples include rapamycin, paclitaxel and E2F Decoy agents. Any of the foregoing can be administered locally or systematically; if locally, certain agents can be contained within the implantable flowable composition.

Cell Source: As described herein, the implantable flowable composition of the present invention comprises cells. Cells can be allogeneic, xenogeneic or autologous. In certain embodiments, a source of living cells can be derived from a suitable donor or multiple donors. In certain other embodiments, a source of cells can be derived from a cadaver or from a cell bank.

In one currently preferred embodiment, cells are endothelial cells. In a particularly preferred embodiment, such endothelial cells are obtained from vascular tissue, preferably but not limited to arterial tissue. As exemplified below, one type of vascular endothelial cell suitable for use is an aortic endothelial cell. Another type of vascular endothelial cell suitable for use is umbilical cord vein endothelial cells. And, another type of vascular endothelial cell suitable for use is coronary artery endothelial cells. Yet other types of vascular endothelial cells suitable for use with the present invention include pulmonary artery endothelial cells and iliac artery endothelial cells.

In another currently preferred embodiment, suitable endothelial cells can be obtained from non-vascular tissue. Non-vascular tissue can be derived from any tubular anatomical structure as described elsewhere herein or can be derived from any non-vascular tissue or organ.

In yet another embodiment, endothelial cells can be derived from endothelial progenitor cells or stem cells; in still another embodiment, endothelial cells can be derived from progenitor cells or stem cells generally. In other preferred embodiments, cells can be non-endothelial cells that are allogeneic, xenogeneic or autologous derived from vascular or non-vascular tissue or organ. The present invention also contemplates any of the foregoing which are genetically altered, modified or engineered.

In a further embodiment, two or more types of cells are co-cultured to prepare the present composition. For example, a first cell can be introduced into the biocompatible implantable material and cultured until confluent. The first cell type can include, for example, smooth muscle cells, endothelial cells, fibroblasts, stem cells, endothelial progenitor cells, cardiomyocytes, a combination of smooth muscle cells and fibroblasts, any other desired cell type or a combination of desired cell types suitable to create an environment conducive to endothelial cell growth. Once the first cell type has reached confluence, a second cell type is seeded on top of the first confluent cell type in, on or within the biocompatible implantable material and cultured until both the first cell type and second cell type have reached confluence. The second cell type may include, for example, endothelial cells or any other desired cell type or combination of cell types. The first and second cell types may include the same cell type derived from two or more different donors or sources. It is contemplated that the first and second cell types may be introduced step wise, or as a single mixture. It is also contemplated that cell density can be modified to alter the ratio of smooth muscle cells to endothelial cells of about 2:1 for an AV graft application, a ratio of about 1:1 for a peripheral bypass application, or another ratio suitable for another clinical application.

To prevent over-proliferation of smooth muscle cells or another cell type prone to excessive proliferation, the culture procedure can be modified. For example, following confluence of the first cell type, the culture can be coated with an attachment factor suitable for the second cell type prior to introduction of the second cell type. Exemplary attachment factors include coating the culture with gelatin to improve attachment of endothelial cells. According to another embodiment, heparin can be added to the culture media during culture of the second cell type to reduce the proliferation of the first cell type and to optimize the desired first cell type to second cell type ratio. For example, after an initial growth of smooth muscle cells, heparin can be administered to control smooth muscle cell growth to achieve a greater ratio of endothelial cells to smooth muscle cells.

In a preferred embodiment, a co-culture is created by first seeding a biocompatible matrix with smooth muscle cells to create vessel structures. Once the smooth muscle cells have reached confluence, endothelial cells are seeded on top of the cultured smooth muscle cells on the implantable material to create a simulated blood vessel. This embodiment can be administered, for example, to an AV graft or peripheral bypass graft according to methods described herein to promote the integration of the prosthetic graft material.

All that is required of the cells of the present composition is that they exhibit one or more preferred phenotypes or functional properties. As described earlier herein, the present invention is based on the discovery that a cell having a readily identifiable phenotype when associated with a preferred biocompatible matrix (described elsewhere herein) can facilitate, restore and/or otherwise modulate endothelial cell physiology and/or luminal homeostasis associated with the treatment of an injured or diseased target vascular endothelium or an injured or diseased target lumen of another tubular anatomical structure.

For purposes of the present invention, one such preferred, readily identifiable phenotype typical of cells of the present invention is an ability to inhibit or otherwise interfere with smooth muscle cell proliferation as measured by the *in vitro* assays described below. This is referred to herein as the inhibitory phenotype.

Another readily identifiable phenotype exhibited by cells of the present composition is that they are anti-thrombotic or are able to inhibit platelet adhesion and aggregation. Anti-thrombotic activity can be determined using an *in vitro* heparan sulfate assay and/or an *in vitro* platelet aggregation assay, described below.

In a typical operative embodiment of the present invention, cells need not exhibit more than one of the foregoing phenotypes. In certain embodiments, cells can exhibit more than one of the foregoing phenotypes.

While the foregoing phenotypes each typify a functional endothelial cell, such as but not limited to a vascular endothelial cell, a non-endothelial cell exhibiting such a phenotype(s) is considered endothelial-like for purposes of the present invention and thus suitable for use with the present invention. Cells that are endothelial-like are also referred to herein as functional analogs of endothelial cells; or functional mimics of endothelial cells. Thus, by way of example only, cells suitable for use with the materials and methods disclosed herein also include stem cells or progenitor cells that give rise to endothelial-like cells; cells that are non-endothelial cells in origin yet perform functionally like an endothelial cell using the parameters set forth herein; cells of any origin which are engineered or otherwise modified to have endothelial-like functionality using the parameters set forth herein.

Typically, cells for use in the present invention exhibit one or more of the aforementioned phenotypes when present in confluent, near-confluent or post-confluent populations and associated with a preferred biocompatible matrix such as those described herein. As will be appreciated by one of ordinary skill in the art, near-confluent, confluent or post-confluent populations of cells are identifiable readily by a variety of techniques, the most common and widely-accepted of which is direct microscopic examination. Others include evaluation of cell number per surface area using standard cell counting techniques such as but not limited to a hemacytometer or coulter counter.

Additionally, for purposes of the present invention, endothelial-like cells include but are not limited to cells which emulate or mimic functionally and phenotypcially near-confluent, confluent and post-confluent endothelial cells as measured by the parameters set forth herein.

Thus, using the detailed description and guidance set forth below, the practitioner of ordinary skill in the art will appreciate how to make, use, test and identify operative embodiments of the implantable flowable composition disclosed herein. That is, the teachings provided herein disclose all that is necessary to make and use the present invention's implantable flowable compositions. And further, the teachings provided herein disclose all that is necessary to identify, make and use operatively equivalent cell-containing compositions. At bottom, all that is required is that such equivalents are effective to treat, manage, modulate or ameliorate luminal injury or disease, such as that associated with vascular intervention or cardiovascular disease as a non-limiting example in accordance with the methods disclosed herein. As will be appreciated by the skilled practitioner, equivalent embodiments of the present composition can be identified using only routine experimentation together with the teachings provided herein.

In certain preferred embodiments, endothelial cells used in the implantable flowable composition of the present invention are isolated from the aorta of human cadaver donors. Each lot of cells can be derived from a single or multiple donors. Each lot is tested extensively for endothelial cell purity, biological function, the presence of bacteria, fungi, known human pathogens and other adventitious agents. The cells are cryopreserved and banked using well-known techniques for later expansion in culture for subsequent formulation in implantable compositions.

Cell Preparation: As stated above, suitable cells can be obtained from a variety of tissue types and cell types. In certain preferred embodiments, human aortic endothelial cells used in the implantable flowable composition are isolated from the aorta of cadaver donors. In other embodiments, porcine aortic endothelial cells (Cell Applications, San Diego, CA) are isolated from normal porcine aorta by a similar procedure used to isolate human aortic endothelial cells. Each lot of cells can be derived from a single or multiple donors and is then tested extensively for endothelial cell viability, purity, biological function, the presence of mycoplasma, bacteria, fungi, yeast, known human pathogens and other adventitious agents. The cells are further expanded, characterized and cryopreserved to form a working cell bank at the third to sixth passage using well-known techniques for later expansion in culture and for subsequent formulation in biocompatible implantable material.

The human or porcine aortic endothelial cells are prepared in T-75 flasks pre-treated by the addition of approximately 15 ml of endothelial cell growth media per flask. Human aortic endothelial cells are prepared in Endothelial Growth Media (EGM-2, Cambrex Biosciences, East Rutherford, NJ). EGM-2 consists of Endothelial Basal Media (EBM-2, Cambrex Biosciences) supplemented with EGM-2 singlequots, which contain 2% FBS. Porcine cells are prepared in EBM-2 supplemented with 5% FBS and 50 µg/ml gentamicin. The flasks are placed in an incubator maintained at approximately 37°C and 5% CO₂ / 95% air, 90% humidity for a minimum of 30 minutes. One or two vials of the cells are removed from the - 160°C to -140°C freezer and thawed at approximately 37°C. Each vial of thawed cells is seeded into two T-75 flasks at a density of approximately 3 x 10³ cells per cm³, preferably, but no less than 1.0 x 10³ and no more than 7.0 x 10³; and the flasks containing the cells are returned to the incubator. After about 8-24 hours, the spent media is removed and replaced with fresh media. The media is changed every two to three days, thereafter, until the cells reach approximately 85-100% confluence preferably, but no less than 60% and no more than 100%. When the implantable flowable composition is intended for clinical application, only antibiotic-free media is used in the post-thaw culture of human aortic endothelial cells and manufacture of the implantable flowable composition of the present invention.

The endothelial cell growth media is then removed, and the monolayer of cells is rinsed with 10 ml of HEPES buffered saline (HEPES). The HEPES is removed, and 2 ml of trypsin is added to detach the cells from the surface of the T-75 flask. Once detachment has occurred, 3 ml of trypsin neutralizing solution (TNS) is added to stop the enzymatic reaction. An additional 5 ml of HEPES is added, and the cells are enumerated using a hemocytometer. The cell suspension is centrifuged and adjusted to a density of, in the case of human cells, approximately 1.75 x 10⁶ cells/ml using EGM-2 without antibiotics, or in the case of porcine cells, approximately 1.50 x 10⁶ cells/ml using EBM-2 supplemented with 5% FBS and 50 µg/ml gentamicin.

Biocompatible Matrix: According to the present invention, one preferred embodiment of implantable flowable composition comprises a biocompatible matrix in the form of a gel, a foam, a suspension, a microcarrier, a microcapsule, a flowable fibrous structure, or other flowable material. The biocompatible matrix is permissive for cell growth and attachment to, on or within the matrix. The biocompatible matrix, when implanted on an exterior surface of a blood vessel for example, can reside at the implantation site for about 7-90 days, preferably at least about 7-14 days, more preferably at least about 14-28 days, most preferably at least about 28-90 days before it bioerodes.

For purposes of the present invention, flowable composition means a composition susceptible to administration using an injection or injection-type delivery device such as, but not limited to, a needle, syringe or a catheter. Other delivery devices which employ extrusion, expulsion or ejection are also contemplated herein. Any non-solid formulation of a biocompatible matrix for use with an injection-type delivery device capable of either endovascular administration by navigating along the interior length of a blood vessel or local percutaneous administration is contemplated herein. A preferred flowable composition is shape-retaining. An implantable flowable composition comprising cells engrafted in a flowable particulate matrix as contemplated herein is formulated for use with any injectable delivery device containing a needle ranging in internal diameter from 22 gauge to 26 gauge, a needle ranging in length from about 1 to 20 mm. A preferred injectable delivery device is capable of delivering about 50 mg of implantable particulate material containing about 1 million cells in about 1 to about 3 ml of media.

According to a currently preferred embodiment of the present invention, the flowable composition comprises a biocompatible particulate matrix such as Gelfoam^{®} particles, Gelfoam^{®} powder, or pulverized Gelfoam^{®} (Pfizer Inc., New York, NY) (hereinafter "Gelfoam particles"), a product derived from porcine dermal gelatin. According to another embodiment, the biocompatible particulate material is Cytodex-3 (Amersham Biosciences, Piscataway, NJ) microcarriers, comprised of denatured collagen coupled to a matrix of cross-linked dextran. According to alternative embodiments, the biocompatible implantable particulate matrix is comprised of modified alginate particles; a biocompatible polymer such as a synthetic polymer degraded by hydrolysis, for example, polyhydroxy acids like polylactic acid, polyglycolic acid and copolymers thereof; polyorthoesters; polyanhydrides; proteins such as gelatin, collagen, fibrin gel; or carbohydrates or polysaccharides such as cellulose and derivatized celluloses, chitosan, alginate, or combinations thereof. A biocompatible matrix is a material that is capable of gradually disappearing over the course of several days, weeks or months after administration of the flowable composition. The rate of degradation depends on the biocompatible matrix chosen and rates of degradation can be modified based on the nature of the treatment and clinical circumstances.

According to another embodiment, the implantable particulate matrix can be a modified particulate matrix. Modifications to the implantable particulate matrix can be selected to optimize and/or to control function of the cells, including the cells' phenotype (e.g., the inhibitory phenotype) as described above, when the cells are associated with the implantable particulate matrix. According to one embodiment, modifications to the implantable particulate matrix include coating the particles with attachment factors or adhesion peptides that enhance the ability of the cells to inhibit smooth muscle cell proliferation, to decrease inflammation, to increase heparan sulfate production, to increase prostacyclin production and/or to increase TGF-β₁ production. Exemplary attachment factors include, for example, fibronectin, fibrin gel, and covalently attached cell adhesion ligands (including for example RGD) utilizing standard aqueous carbodiimide chemistry. Additional cell adhesion ligands include peptides having cell adhesion recognition sequences, including but not limited to: RGDY, REDVY, GRGDF, GPDSGR, GRGDY and REDV.

According to another embodiment, the implantable particulate matrix is a particle other than Gelfoam. Additional exemplary particulate matrices include, for example, fibrin gel, alginate, polystyrene sodium sulfonate microcarriers, collagen coated dextran microcarriers, PLA/PGA and pHEMA/MMA copolymers (with polymer ratios ranging from 1-100% for each copolymer). According to a preferred embodiment, these additional particulate matrices are modified to include attachment factors or adhesion peptides, as recited and described above. Exemplary attachment factors include, for example, gelatin, collagen, fibronectin, fibrin gel, and covalently attached cell adhesion ligands (including RGD) utilizing standard aqueous carbodiimide chemistry. Additional cell adhesion ligands include peptides having cell adhesion recognition sequences, including but not limited to: RGDY, REDVY, GRGDF, GPDSGR, GRGDY and REDV.

According to another embodiment, the implantable particulate matrix is physically modified to improve cell attachment. According to one embodiment, the implantable particulate matrix is cross-linked to enhance its mechanical properties and to improve its cell attachment and growth properties. According to a preferred embodiment, an alginate particle is first cross linked using calcium sulfate followed by a second cross linking step using calcium chloride and routine protocols. According to another embodiment, a source of heparin or heparan sulfate, for example heparin-sepharose, is incorporated into the matrix prior to cell culture.

It is contemplated that the implantable flowable composition comprising a biocompatible particulate matrix can be delivered using, preferably, a 22gauge to 26gauge internal diameter needle. Accordingly, particles that form such a matrix preferably are of a diameter capable of passing through the aperture of a suitable needle, as defined herein. According to a preferred embodiment, the particles of such a matrix have a diameter of about 20 µm to about 1000 µm, with a preferred diameter of about 100 µm to about 500 µm, most preferably a diameter of about 200 µm.

Each particle of a preferred particulate matrix should have at least one cell adhered to its surface, preferably more than one cell. Accordingly, each particle should have a diameter larger than the spread diameter of the chosen cell type. For example, endothelial cells have a spread diameter of approximately 18 µm; to encourage attachment of more than one endothelial cell to a particle, each particle should be at least 20 µm in diameter.

Preferred Culture Tubes: In certain embodiments contemplated herein, approximately 50-60 mg of Gelfoam particles are placed into individual 50 mL tubes (Evergreen, Los Angeles, CA) with 0.2 µm filter caps. In order to reduce the number of particles lost during media changes, culture tubes can be modified by, for example, adding a filter to the cap area or to an interior portion of the culture tube to prevent aspiration or other unintended removal of particles during expulsion of the media, adding a partition to the culture tube to create separate portions for media and particles with a means of fluid communication between the portions capable of passing media but not particles, or adding a spout on the bottom of the culture tube that can be opened and the media poured off without disturbing the particles.

Cell Seeding of Implantable Material: Prior to cell seeding, the particles are prepared by the addition of 70% ethanol followed by several rinses in PBS or HEPES. The particles are then re-hydrated in EGM-2 without antibiotics at approximately 37°C and 5% CO₂ / 95% air for 12 to 24 hours. Aliquots of approximately 50-60 mg of particles are then removed from their re-hydration containers and placed in individual tissue culture dishes. The aliquot of particles is seeded at a preferred density of 2 x 10³ to 2 x 10⁴ cells per mg particles. The cells-matrix mixture is pipetted up and down several times to form a uniform suspension. The tubes are then incubated at 37°C, 5% CO₂, 90% humidity with periodic agitation for 3 to 4 hours to facilitate cell attachment. An additional 9 mL of EGM-2 is then added per tube (final media volume = 10 mL), resulting in a final particle volume of about 5 mg particles / mL media.

The media is changed every two to three days, thereafter, until the cells have reached confluence. The cells in one preferred embodiment are preferably passage 6, but cells of fewer or more passages can be used.

Cell Growth Curve and Confluence: A sample of implantable flowable composition is removed on or around days 3 or 4, 6 or 7, 9 or 10, and 12 or 13, the cells are counted and assessed for viability, and a growth curve is constructed and evaluated in order to assess the growth characteristics and to determine whether near-confluence, confluence or post-confluence has been achieved. A representative growth curve from a preparation of implantable flowable composition comprising porcine aortic endothelial cell implanted lots is presented in FIG. 1. In this example, the implantable material is in a particulate form. Generally, one of ordinary skill will appreciate the indicia of acceptable cell growth at early, mid- and late time points, such as observation of an increase in cell number at the early time points (when referring to FIG. 1, between about days 3 to 10), followed by a near-confluent phase (when referring to FIG. 1, between about days 10 to 13), followed by a plateau in cell number once the cells have reached confluence (when referring to FIG. 1, between about days 13 to 15) and maintenance of the cell number when the cells are post-confluent (when referring to FIG. 1, between about days 15 to 17). For purposes of the present invention, cell populations which are in a plateau for at least 72 hours are preferred.

Cell counts are achieved by complete digestion of the aliquot of implantable flowable composition with a solution of 0.8 mg/ml collagenase in a trypsin-EDTA for Gelfoam particles or a solution of dextranase and trypsin-EDTA for Cytodex-3 particles. After measuring the volume of the digested implantable flowable composition, a known volume of the cell suspension is diluted with 0.4% trypan blue (4:1 cells to trypan blue) and viability assessed by trypan blue exclusion. Viable, non-viable and total cells are enumerated using a hemacytometer. Growth curves are constructed by plotting the number of viable cells versus the number of days in culture. Cells are shipped and implanted after reaching confluence.

For purposes of the present invention, confluence is defined as the presence of at least about 8 x 10³ cells/mg of biocompatible particles, preferably about 7 x 10⁵ to about 1 x 10⁶ total cells per aliquot of 50-70 mg particles with viability of preferably at least about 90% but no less than about 80%. Cell viability is at least about 90% preferably but no less than about 80%. If the cells are not confluent by day 12 or 13, the media is changed, and incubation is continued for an additional day. This process is continued until confluence is achieved or until about 14 days post-seeding. If the cells are determined to be confluent after performing in-process checks, a final media change is performed. This final media change is performed using EGM-2 without phenol red and without antibiotics. Immediately following the media change, the tubes are fitted with sterile plug seal caps for shipping.

Evaluation of Functionality: For purposes of the invention described herein, the implantable flowable composition is further tested for indicia of functionality prior to implantation. For example, conditioned media are collected during the culture period to ascertain levels of heparan sulfate, transforming growth factor-β₁ (TGF-β₁), basic fibroblast growth factor (b-FGF), and nitric oxide which are produced by the cultured endothelial cells. In certain preferred embodiments, the implantable flowable composition can be used for the purposes described herein when total cell number is at least 2, preferably at least 8 x 10³ cells/cm³; percentage of viable cells is at least 80-90%, preferably ≥90%, most preferably at least 90%. Heparan sulfate in conditioned media is at least 0.5-1.0, preferably at least 1.0 microg/10⁶ cell/day. TGF-β₁ in conditioned media is at least 200-300, preferably at least 300 picog/ml/day; b-FGF in conditioned media is below about 200 picog/ml, preferably no more than about 400 picog/ml.

Heparan sulfate levels can be quantitated using a routine dimethylmethylene blue-chondroitinase ABC digestion spectrophotometric assay. Total sulfated glycosaminoglycan (GAG) levels are determined using a dimethylmethylene blue (DMB) dye binding assay in which unknown samples are compared to a standard curve generated using known quantities of purified chondroitin sulfate diluted in collection media. Additional samples of conditioned medium are mixed with chondroitinase ABC to digest chondroitin and dermatan sulfates prior to the addition of the DMB color reagent. All absorbances are determined at the maximum wavelength absorbance of the DMB dye mixed with the GAG standard, generally around 515-525 nm. The concentration of heparan sulfate per 10⁶ cells per day is calculated by subtracting the concentration of chondroitin and dermatan sulfate from the total sulfated glycosaminoglycan concentration in conditioned medium samples. Chondroitinase ABC activity is confirmed by digesting a sample of purified chondroitin sulfate. Conditioned medium samples are corrected appropriately if less than 100% of the purified chondroitin sulfate is digested. Heparan sulfate levels may also be quantitated using an ELISA assay employing monoclonal antibodies.

TGF-β₁ and b-FGF levels can be quantitated using an ELISA assay employing monoclonal or polyclonal antibodies, preferably polyclonal. Control collection media can also be quantitated using an ELISA assay and the samples corrected appropriately for TGF-β₁ and b-FGF levels present in control media.

Nitric oxide (NO) levels can be quantitated using a standard Griess Reaction assay. The transient and volatile nature of nitric oxide makes it unsuitable for most detection methods. However, two stable breakdown products of nitric oxide, nitrate (NO₃) and nitrite (NO₂), can be detected using routine photometric methods. The Griess Reaction assay enzymatically converts nitrate to nitrite in the presence of nitrate reductase. Nitrite is detected colorimetrically as a colored azo dye product, absorbing visible light in the range of about 540 nm. The level of nitric oxide present in the system is determined by converting all nitrate into nitrite, determining the total concentration of nitrite in the unknown samples, and then comparing the resulting concentration of nitrite to a standard curve generated using known quantities of nitrate converted to nitrite.

The earlier-described preferred inhibitory phenotype is assessed using the quantitative heparan sulfate, TGF-β₁, NO and/or b-FGF assays described above, as well as quantitative *in vitro* assays of smooth muscle cell growth and inhibition of thrombosis as follows. For purposes of the present invention, implantable flowable composition is ready for implantation when one or more of these alternative *in vitro* assays confirm that the implantable flowable composition is exhibiting the preferred inhibitory phenotype.

To evaluate inhibition of smooth muscle cell growth *in vitro,* the magnitude of inhibition associated with cultured endothelial cells is determined. Porcine or human aortic smooth muscle cells are sparsely seeded in 24 well tissue culture plates in smooth muscle cells growth medium (SmGM-2, Cambrex BioScience). The cells are allowed to attach for 24 hours. The medium is then replaced with smooth muscle cell basal media (SmBM) containing 0.2% FBS for 48-72 hours to growth arrest the cells. Conditioned media is prepared from post-confluent endothelial cell cultures, diluted 1:1 with 2X SMC growth media and added to the cultures. A positive control for inhibition of smooth muscle cell growth, for example, heparin, is included in each assay. After three to four days, the number of cells in each sample is enumerated using a Coulter Counter. The effect of conditioned media on smooth muscle cell proliferation is determined by comparing the number of smooth muscle cells per well immediately before the addition of conditioned medium with that after three to four days of exposure to conditioned medium, and to control media (standard growth media with and without the addition of growth factors). The magnitude of inhibition associated with the conditioned media samples are compared to the magnitude of inhibition associated with the positive control. According to a preferred embodiment, the implantable flowable composition is considered inhibitory if the conditioned media inhibits about 20% of what the heparin control is able to inhibit.

To evaluate inhibition of thrombosis *in vitro,* the level of heparan sulfate associated with the cultured endothelial cells is determined. Heparan sulfate has both anti-proliferative and anti-thrombotic properties. Using either the routine dimethylmethylene blue-chondroitinase ABC spectrophotometric assay or an ELISA assay, both assays are described in detail above, the concentration of heparan sulfate per 10⁶ cells is calculated. The implantable flowable composition can be used for the purposes described herein when the heparan sulfate in the conditioned media is at least 0.5-1.0, preferably at least 1.0 microg/10⁶ cells/day.

Another method to evaluate inhibition of thrombosis involves determining the magnitude of inhibition of platelet aggregation *in vitro* associated with platelet rich-plasma. Porcine plasma is obtained by the addition of sodium citrate to porcine blood samples at room temperature. Citrated plasma is centrifuged at a gentle speed, to draw red and white blood cells into a pellet, leaving platelets suspended in the plasma. Conditioned media is prepared from post-confluent endothelial cell cultures and added to aliquots of the platelet-rich plasma. A platelet aggregating agent (agonist) is added to the plasma as control. Platelet agonists commonly include arachidonate, ADP, collagen, epinephrine, and ristocetin (available from Sigma-Aldrich Co., St. Louis, MO). An additional aliquot of plasma has no platelet agonist or conditioned media added, to assess for baseline spontaneous platelet aggregation. A positive control for inhibition of platelet aggregation is also included in each assay. Exemplary positive controls include aspirin, heparin, abciximab (ReoPro^{®}, Eli Lilly, Indianapolis, IN), tirofiban (Aggrastat^{®}, Merck & Co., Inc., Whitehouse Station, NJ) or eptifibatide (Integrilin^{®}, Millennium Pharmaceuticals, Inc., Cambridge, MA). The resulting platelet aggregation of all test conditions are then measured using an aggregometer. The aggregometer measures platelet aggregation by monitoring optical density. As platelets aggregate, more light can pass through the specimen. The aggregometer reports results in "platelet aggregation units," a function of the rate at which platelets aggregate. Aggregation is assessed as maximal aggregation at 6 minutes after the addition of the agonist. The effect of conditioned media on platelet aggregation is determined by comparing baseline platelet aggregation before the addition of conditioned medium with that after exposure of platelet-rich plasma to conditioned medium, and to the positive control. Results are expressed as a percentage of the baseline. The magnitude of inhibition associated with the conditioned media samples are compared to the magnitude of inhibition associated with the positive control. According to a preferred embodiment, the implantable flowable composition is considered inhibitory if the conditioned media inhibits about 20% of what the positive control is able to inhibit.

Transport Container: Immediately following the media change, the implantable flowable composition is packaged for shipping. According to one embodiment, the same culture tubes used for culturing the cells on the implantable material are fitted with sterile plug seal caps for shipping. To decrease the risk of decanting particles and/or cells during the final rinse, the shipping container can be modified to include, for example, a filter or other entrapment device with a pore size capable of passing media and rinse solution, but not capable of decanting particles and/or cells.

If the implantable flowable composition is shipped in media containing serum, just prior to implantation the implantable flowable composition is rinsed, preferably within the culture tube. The filter or other entrapment means is then removed from the transport container and the cell engrafted particles are drawn into a syringe for delivery. The excess rinse solution is expelled from the syringe and a needle, catheter or other delivery device is attached to the syringe for delivery to the treatment site. The flowable composition is then delivered to the patient, for example, according to one of the exemplary administration methods discussed below. If the implantable flowable composition is transported in serum-free media, it is not necessary to perform the final rinse procedure at the clinical site.

According to an alternative embodiment, the implantable flowable composition is drawn from the culture tube into a syringe, along with about 1 to 20 ml of media, or a sufficient volume of media for transport and storage, the syringe is capped and the material is shipped in the sealed syringe. At the site of implantation, excess media is expelled from the syringe. A needle, catheter or other delivery device is then attached to the syringe for delivery to the treatment site. According to this embodiment, the flowable composition is delivered to the patient directly from the transport syringe.

The implantable composition of the present invention can be supplied in final product containers, including, for example, 50 ml or 60 ml sealed tissue culture containers modified with filter caps or pre-loaded syringes, each preferably containing about 50-60 mg of particulate material engrafted with about 7 x 10⁵ to about 1 x 10⁶ total endothelial cells in about 45-60 ml, preferably about 50 ml, endothelial growth medium per aliquot. The total cell load per patient will be preferably approximately 0.6-12 x 10⁴ cells per kg body weight, but no less than 2 x 10³ and no more than 2 x 10⁵ cells per kg body weight.

As contemplated herein, the material of the present invention comprises cells, preferably vascular endothelial cells, which are preferably about 90% viable at a density of preferably about 1.4-2.1 x 10⁴ cells/mg particles in one preferred embodiment, and when confluent or near-confluent, produce conditioned media containing heparan sulfate at least about 0.5-1.0, preferably at least about 1.0 microg/10⁶ cell/day. TGF-β₁ in conditioned media is at least about 200-300, preferably at least about 300 picog/ml/day; b-FGF in conditioned media is below about 200 picog/ml, preferably no more than about 400 picog/ml.

According to another embodiment, one or more additional substances are added to the flowable composition prior to administration. Such substances include, but are not limited to, anti-inflammatory agents, glycosaminoglycans, prostaglandins, prostanoids, cytokines including but not limited to TGF and VEGF, angiotensin and related compounds, tyrosine kinase inhibitors, immunosuppressants, vitamins, glucocorticoids, anti-oxidants, free radical scavengers, peptide hormones, angiogenic and angiogenic-inhibitory factors.

Carriers: Optionally, according to one embodiment, the implantable flowable composition includes a carrier fluid. A preferred carrier fluid is cell growth media for facilitating administration. Laboratory simulated administrations of the implantable flowable composition indicate that a carrier is not necessary to preserve cell integrity when handling flowable compositions. It is unexpected that cell formulations such as those described herein can be free of carriers or other agents which are typically employed to preserve cell integrity; or agents which are typically employed to improve handling and reduce shear force-induced disruption of non-solid or flowable formulations.

However, a carrier can serve to improve cell confluency and viability within the flowable composition, for example, during cell culture, including during expulsion of conditioned media and introduction of new media, during manipulation of the implantable flowable composition prior to transport, while the material is drawn into the delivery syringe, and/or during expulsion of the material from the syringe and delivery of the material into the perivascular space or other target site.

The carrier can be introduced into the implantable material at a variety of points during the cell culture procedure. For example, the carrier may be added to the particulate matrix at the time of particle hydration (prior to cell seeding), just prior to or at the time of cell seeding, and/or added incrementally during scheduled media changes in the cell culture procedure. By introducing the carrier earlier in the cell culture, those cells that are possibly adversely affected can be sloughed off and the remaining cells can proliferate to maintain a sufficient cell population.

Alternatively, the carrier can be introduced during the last media change, when the cells are confluent or near-confluent, and just prior to implantation. However, it has been observed that addition of an undiluted glycerol carrier just prior to administration, can result in a shock to the cells perhaps, suffocating the cells sufficiently to reduce the desired cell viability and efficacy below acceptable levels. Based on these observations, it is possible that adding a highly viscous carrier fluid, such as glycerol, to the flowable composition all at once can adversely affect the cells and should be avoided.

In order to evaluate the efficacy of other candidate carrier fluids, initial trials will be conducted using a high particle to carrier ratio to determine the minimum amount of carrier necessary to achieve the desired benefits. The desired benefits to be evaluated include, for example, improved handling and improved flowability of composition while maintaining cell viability and efficacy. Additionally, studies will be conducted to test the ability to transform a planar form of implantable composition into an implantable particulate flowable composition, without affecting cell viability or function. For example, initial test solutions will include only about a 0.1-1% solution of a carrier to 50 mg of particles. This concentration will be increased incrementally up to a maximum of about a 10% solution of a carrier to 50 mg of particles.

Subsequent trials will be conducted wherein a carrier fluid is added incrementally over the entire culture course, beginning at the time of particle hydration or cell seeding, so that by the time the cells in the implantable flowable composition have reached confluence and are ready to be shipped and/or administered to a patient, the concentration of the carrier fluid in the implantable material has increased from the lower tolerable ratio for early cell attachment to a higher optimal ratio of carrier fluid to particles. For example, according to an exemplary protocol, a solution of 0.1% carrier will be added to the particulate material at the time of initial hydration and at each subsequent media change, up to a final solution of 1% carrier. It is expected that some cells will be lost at each introduction of the carrier fluid, but that most cells will incorporate themselves into the implantable flowable composition conditioned with a carrier and will be able to grow to confluence or near-confluence within this environment.

Carriers include, without limitation, diluted glycerol, alginate (preferably about 1% alginate solution), dextran or dextrose sugars (preferably about 1-10% dextran or dextrose solution), other sugars including, for example, glucose, sucrose, and fructose, starches (preferably about 6% hydroxyethyl starch solution), gelatin (preferably about 1-2% gelatin solution), endothelial growth media, endothelial basal media, other cell growth media or neutral buffered saline. Additional percent solution ranges are contemplated but not expressly defined herein. Variations are expected depending, in part, on the type of biocompatible matrix used, the type of cell engrafted thereto, and the mode of administration chosen for delivery. For example, the cell culture media used for initial hydration and subsequent media changes can include about 0.5% up to about 10% by volume carrier fluid, depending on the chosen carrier. In general, the selected carrier should be non-cytotoxic at the dosage and concentration employed and sufficiently permeable to allow air and nutrients to flow in (to support cell growth) and out (to remove cell waste products) of the implantable composition.

Effect of Undiluted Glycerol Carrier on Cell Viability: Porcine aortic endothelial cells were seeded on 100 mg of Gelfoam particles and allowed to proliferate to confluence. The particulate flowable composition was harvested, placed into 50 ml tubes and mixed with 3 ml aliquots of undiluted glycerol before being placed in syringes attached to 24, 26, or 27 gauge needles. The contents of all syringes were slowly expelled through the attached needles and collected in 50 ml tubes. Only 0-5% of all cells visible after expulsion were viable (95-100% of the cells were dead), compared to 86-93% viability for cells in the same trial without added glycerol, dramatically illustrating the detrimental effect on cell viability of this carrier liquid when added undiluted as a single dose to confluent cells. It was not determined whether the use of this carrier fluid was beneficial to maintaining confluence of the cell monolayers.

Planar Material Extrusion and Modification: According to another embodiment, a solid, semi-solid, or large diameter composition is modified to form particles capable of being delivered through an injectable delivery device after the cells have sufficiently engrafted to the implantable composition and reached confluence. According to one embodiment, the cells are cultured in the presence of a carrier fluid, as described in greater detail above, to maintain cell confluency and integrity during material modification.

According to one embodiment once the cells have reached confluence on a planar form of implantable composition, the composition is transferred to a syringe. In order to accommodate the planar form, the syringe can have no needle or can have a large bore needle. The syringe sucks up the flexible planar form and then, under pressure, the cell-engrafted composition is extruded through the opening of the syringe to form a non-planar flowable composition. In order to obtain a preferred particle size, several passages through the syringe may be necessary. For example, the material can be passed through the syringe first without a needle, followed by passage through a large bore needle and then passage through smaller bore needles until the material has reached the desired particle size and flowability. Multiple passages and incremental modifications to particle size are desirable to reduce the amount of damage to the cells and to maintain cell confluency during such a material modification.

Surgical Sealants: In certain other embodiments, the flowable composition of the present invention can additionally serve as an anastomotic sealant specifically or surgical sealant generally. In such a dual purpose embodiment, the composition is also effective to seal the juncture of two or more tubular structures or to seal a void in a tubular structure when contacted with an exterior surface of the structure(s), or applied in an arc on an exterior surface, or applied circumferentially. Such a sealant can eliminate a requirement for sutures which can further damage vascular tissue, for example, and contribute to luminal endothelial trauma. Such a sealant can also provide additional stability in the vicinity of an anastomosis thereby reinforcing any suture repair. All that is required is that the sealant-type functions or properties of this dual purpose composition do not interfere with or impair coincident expression of the cells' desired phenotype and the cell-based functionality of the composition.

For purposes of certain sealant embodiments, the flowable composition comprises a biocompatible substrate which itself comprises a component having sealant properties, such as but not limited to a fibrin network, while also having the requisite properties for supporting endothelial or endothelial-like cell populations. Also, the biocompatible substrate *per se* can have both sealant properties as well as those required to support a population of cells. In the case of other embodiments, sealant functionality can be contributed, at least in part, by the cells. For example, it is contemplated that cells associated with the composition produce a substance that can modify a substrate, such that the substrate acquires sealant properties, while also exhibiting/maintaining their requisite cellular functionality. Certain cells can produce this substance naturally while other cells can be engineered to do so.

Shelf-Life of Implantable Flowable Composition: The implantable flowable composition comprising a confluent, near-confluent or post-confluent population of cells can be maintained at room temperature in a stable and viable condition for at least two weeks. Preferably, such implantable flowable composition is maintained in about 45-60 ml, more preferably about 50 ml, transport media with or without additional FBS. Transport media comprises EGM-2 media without phenol red. FBS can be added to the volume of transport media up to about 10% FBS, or a total concentration of about 12% FBS. However, because FBS must be removed from the implantable flowable composition prior to implantation, it is preferred to limit the amount of FBS used in the transport media to reduce the length of rinse required prior to implantation.

Cryopreservation of Implantable Flowable Composition: The implantable flowable composition comprising a confluent, near-confluent or post-confluent population of cells can be cryopreserved for storage and/or transport to the clinic without diminishing its clinical potency or integrity upon eventual thaw. Preferably, the implantable flowable composition is cryopreserved in a 15 ml cryovial (Nalgene^{®}, Nalge Nunc Int'1, Rochester, NY) in a solution of about 5 ml CryoStor CS-10 solution (BioLife Solutions, Oswego, NY) containing about 10% DMSO, about 2-8% Dextran and about 50-75% FBS. Cryovials are placed in a cold isopropanol water bath, transferred to an -80°C freezer for 4 hours, and subsequently transferred to liquid nitrogen (-150 to -165°C).

Cryopreserved aliquots of the implantable flowable composition are then slowly thawed at room temperature for about 15 minutes, followed by an additional approximately 15 minutes in a room temperature water bath. The material is then washed about 3 times in about 15 ml wash media. Wash media comprises EBM without phenol red and with or without 50 µg/ml gentamicin. The first two rinse procedures are conducted for about 5 minutes at room temperature. The final rinse procedure is conducted for about 30 minutes at 37°C in 5% CO₂.

Following the thaw and rinse procedures, the cryopreserved material is allowed to rest for about 48 hours in about 10 ml of recovery solution. For porcine endothelial cells, the recovery solution is EBM-2 supplemented with 5% FBS and 50 µg/ml gentamicin at 37°C in 5% CO₂. For human endothelial cells, the recovery solution is EGM-2 without antibiotics. Further post-thaw conditioning can be carried out for at least another 24 hours prior to use and/or packaging for storage or transport.

Loading and Uptake in a Delivery Device: Aliquots of flowable composition are packaged and transported in culture tubes or syringes, as described in greater detail above, in about 45-60 ml, preferably about 50 ml, of transport media, with or without serum, to support the cells up to 14 days without media change. Prior to implantation, excess media is decanted and, if serum was present in the transport media, the implantable flowable composition rinsed several times to remove any remaining serum. Because certain preparations of particulate forms of implantable flowable composition are prone to separation and, therefore, loss of cell confluence, the composition should remain within the transport container during the final rinse procedure. Additionally, modifications to the transport container, including but not limited to filters and/or separate media compartments, are preferred to maintain implantable flowable composition integrity during manipulations.

After several rinses of the implantable flowable composition, about 1-3 ml of rinse solution remains on top of the implantable flowable composition to facilitate uptake of the material into the delivery device, for example, a syringe. The delivery device is then manipulated to draw the implantable flowable composition into the delivery device, with care being taken to minimize disruption of the confluent cell layer. According to another embodiment, a material loading device, such as a funnel-shaped interface between the opening of the transport container and the delivery syringe, is used to transfer the material to the delivery device with reduced cell disruption. Following transfer of the material to the delivery device, some of the remaining liquid, about 1 ml, is expelled out of the delivery device to prime the delivery device and to fill the void volume. Approximately 0-2 ml of rinse solution remains in the implantable flowable composition delivered to the patient. The implantable flowable composition is now ready for delivery to the treatment site.

Needle Passage: To demonstrate the utility of the present invention, one preferred particulate implantable flowable composition (HAE engrafted in Gelfoam particles) was passed through the aperture of a 22-gauge needle (internal diameter = 0.016 inches). Alternatively, another preferred composition (PAE engrafted in Gelfoam particles) was passed through the aperture of a needle in the range of about a 21-gauge needle (internal diameter = 0.019 inches) to a 28-gauge needle (internal diameter = 0.007 inches).

As demonstrated below in Table 1, passage of an embodiment of the present invention through a needle with an internal diameter within these ranges does not adversely affect the cell number, viability or functionality of the needle-passaged cells. It is unexpected that a cellular preparation can be taken up and discharged from apertures ranging from 21-gauge (internal diameter = 0.019 inches) to 30-gauge (internal diameter = 0.006 inches), preferably 24-gauge (internal diameter = 0.012 inches), without consequence or compromise in confluence or functionality. It was also unexpected that the cells would perform so well after passage through needles with only cell growth media as a carrier fluid.

As described below, cells were mixed with the particulate matrix and allowed to proliferate to confluence. Three days post confluence, the resulting flowable composition was passed through a sterile needle with an internal diameter in the range of about 0.007 inches to about 0.018 inches, collected, and allowed to recover for a 48 hour period in Endothelial Growth Medium-2 (EGM-2). Following the recovery period, the media was conditioned for 24 hours. The conditioned media of the post-passage cells was then evaluated. The post-passage conditioning media was evaluated for acceptable levels of basic fibroblast growth factor (b-FGF), heparan sulfate (HS) and transforming growth factor-β₁ (TGF-β₁) production. Additionally, a smooth muscle cell assay was conducted to show the inhibition of smooth muscle cell proliferation by the media. Assay results of needle-passaged composition were compared to samples which have not been passed through a needle.

### Table 1

Passage Through 22g Needle

| **Assay Results** | **22g Needle** | **No Needle** |
|---|---|---|
| Cell Count & Viability | 6.4E+6 @ 88.3% | 7.95E+6 @ 90.8% |
| µg HS/10⁶ cells | 1.7 | 1.0 |
| pg TGF-β₁/10⁶ cells | 488.6 | 575.3 |
| pg bFGF/10⁶ cells | 227.6 | 229.2 |
| Smooth Muscle Cell Inhibition | 75% | 100% |
| (PCCM/Heparin Inhibition Ratio) | | |

Passage Through 24g Needle

| **Assay Results** | **24g Needle** | **No Needle** |
|---|---|---|
| Cell Count & Viability | 1E+6 @ 86% | 2.78E+6 @ 92.6% |
| µg HS/10⁶ cells | 9.33 | 8.6 |
| pg TGF-β₁/10⁶ cells | 545.7 | 571.4 |
| pg bFGF/10⁶ cells | 2491 | 744 |
| Smooth Muscle Cell Inhibition | 65% | 45% |
| (PCCM/Heparin Inhibition Ratio) | | |

Catheter Passage: In another demonstration of the unexpected properties of the flowable compositions of the present invention, a preferred formulation was loaded into and administered through an injection and/or a penetration means, for example, a needle catheter (Table 2). In one study, the needle catheter was a 6 French catheter incorporating a thin-walled Nitinol needle (outer diameter=24-gauge; interior diameter=22-gauge) (TransVascular Corp., Palo Alto, CA).

Passage of the flowable composition through a needle catheter with an internal diameter within this range did not adversely affect the cell number, viability or biological output of the cells post-passage. According to a preferred embodiment, cells were seeded on the particulate matrix and allowed to proliferate to confluence. Three days post-confluence, the flowable composition was passed through a sterile needle catheter with an internal diameter in the range of about 0.007 inches to about 0.018 inches, collected, and allowed to recover for a 24 hour period in Endothelial Growth Medium-2 (EGM-2). Following the recovery period, the media was conditioned for 24 hours. The conditioned media of the needle-passaged cells was then evaluated. The post-passage conditioning media was evaluated for acceptable levels of basic fibroblast growth factor (bFGF), heparan sulfate (HS) and transforming growth factor-β₁ (TGF-β₁) production. Additionally, a smooth muscle cell assay was conducted to show the inhibition of smooth muscle cell proliferation by the media. Assay results of needle-passaged cells were compared to the results of cell engrafted particulate material samples which had not been passed through a needle catheter (see Table 2).

### Table 2

Passage Through Needle Catheter

| **Assay Results** | **Needle Catheter** | **No Needle Catheter** |
|---|---|---|
| Cell Count & Viability | 7.9E+6 @ 91.2% | 9.9E+6 @ 91% |
| µg HS/10⁶ cells | 1.1 | 1.0 |
| TGF-β₁ pg/mL | 360 | 336 |
| bFGF pg/mL | 58 | 61.7 |
| Smooth Muscle Cell Inhibition | 92% | 85% |
| (PCCM/Heparin Inhibition Ratio) | | |

Endovascular Administration: The flowable composition can be administered intraluminally, i.e. endovascularly. For example, the composition can be delivered by any device able to be inserted within the blood vessel to be treated. Endoscopic guidance systems may be used to locate the delivery device at the site of administration, including, for example, intravascular ultrasound (IVUS), color Doppler ultrasound, duplex ultrasound, other routine ultrasound, angiography, magnetic resonance angiography (MRA), magnetic resonance imaging (MRI), CT scanning, fluoroscopy to identify the location of a stent and/or other endoscopic guidance systems known in the field. Additionally, the site of administration may be located using tactile palpation. Endovascular delivery of the formulation to the site of administration can occur alone or in combination and prior to, at the time of, or following another endovascular procedure, such as balloon angioplasty or implantation of a stent or other device.

In one instance, the intraluminal delivery device is equipped with a traversing or penetrating device which penetrates the luminal wall of a blood vessel to reach a non-luminal surface of a blood vessel. The flowable composition is then deposited on a non-luminal surface of a blood vessel at, adjacent to or in the vicinity of an injured or diseased target site.

It is contemplated herein that a non-luminal, also termed an extraluminal, surface can include an exterior or perivascular surface of a vessel, or can be within the adventitia, media, or intima of a blood vessel, for example. For purposes of this invention, non-luminal or extraluminal is any surface except an interior surface of the lumen.

The penetrating devices contemplated herein can permit, for example, a single point of delivery or a plurality of delivery points arranged in a desired geometric configuration to accomplish delivery of the flowable composition to a non-luminal surface of a blood vessel without disrupting an injured or diseased target site. A plurality of delivery points can be arranged, for example, in a circle, a bulls-eye, or a linear array arrangement to name but a few. The penetrating device can also be in the form of a stent perforator, such as but not limited to, a balloon stent including a plurality of delivery points.

Percutaneous Administration: For purposes of the present invention generally, administration of flowable composition is localized to a site at, adjacent to or in the vicinity of a site in need of treatment. The site of deposition of the implantable flowable composition is extraluminal. As contemplated herein, localized, extraluminal deposition can be accomplished percutaneously as follows.

Flowable composition can be delivered percutaneously using a needle, catheter or other suitable delivery device. The flowable composition can be delivered percutaneously coincident with use of a guidance method to facilitate delivery to the site in need of treatment. The guidance step is optional. Endoscopic guidance systems can be used to locate the site of extraluminal administration, including, for example, intravascular ultrasound (IVUS), color Doppler ultrasound, duplex ultrasound, other routine ultrasound, angiography, magnetic resonance angiography (MRA), magnetic resonance imaging (MRI), CT scanning, fluoroscopy to identify the location of a stent and/or other endoscopic guidance systems known in the field. Additionally, the site of administration can be located using tactile palpation. Upon entry into the perivascular space, the clinician deposits the flowable composition on an extraluminal site at, adjacent or in the vicinity of the site in need of treatment. The guiding or identifying step is optionally performed and not required to practice the methods of the present invention.

The implantable flowable composition may be delivered locally to a surgically-exposed extraluminal site at, adjacent to or in the vicinity of a site in need of treatment. In this case delivery is guided and directed by direct observation of the site in need of treatment. Also in this case, delivery can be aided by coincident use of an identifying step as described above. Again, the identifying step is optional.

Site of Administration: A penetrating device may be inserted via the interior luminal surface of a blood vessel using an endovascular delivery device or through the surrounding tissue in a percutaneous delivery. Administration can be directed to a location proximal to, distal to, or at an injured or diseased target site. In some clinical subjects, insertion of the penetrating device at an injured or diseased target site could disrupt the injured or diseased target site. Accordingly, in such subjects, care should be taken to insert the penetrating device at a location a distance from an injured or diseased target site, preferably a distance determined by the clinician governed by the specific circumstances at hand.

Preferably, flowable composition is deposited on a perivascular surface of a blood vessel, either at, adjacent or in the vicinity of an injured or diseased target site to be treated. The composition can be deposited in a variety of locations relative to an injured or diseased target site, for example, at an injured or diseased target site, adjacent an injured or diseased target site, for example, upstream of an injured or diseased target site, on an opposing exterior vessel surface from an injured or diseased target site. An adjacent site may be within 2 mm to 20 mm of the site of an injured or diseased target site. A deposition site may be within 21 mm to 40 mm; in yet another preferred embodiment, a deposition site is within 41 mm to 60 mm.
In another preferred embodiment, a deposition site is within 61 mm to 100 mm. Alternatively, an adjacent site is any other clinician-determined adjacent location where the deposited composition is capable of exhibiting a desired effect.

An optional planar administration area may be created within an extraluminal target site prior to administration of the implantable flowable composition. A planar administration area is an area prepared to accept a volume of implantable flowable composition and can be created using, for example, blunt dissection, balloon dissection, fluid dissection, or another dissection technique known in the field. The administration area can be created using an endovascular or perivascular dissection device. The implantation of flowable composition at a target site is facilitated by creation of an administration area. An administration area is not required to practice the present invention.

It is contemplated that the implantable flowable composition can be administered in a variety of configurations at the site of administration. For example, the implantable flowable composition can be administered in a linear application, parallel to the direction of blood flow; in a circumferential application, perpendicular to the direction of blood flow; or in a mass at the site of administration. It is also contemplated that the foregoing dissection step and delivery of flowable composition can take place concurrently or sequentially. For example, the implantable flowable composition can itself be used to accomplish fluid dissection if delivered under pressure. However, this method of dissection risks tissue trauma created by pressurized delivery of the flowable composition and could disrupt cell confluency by pressurized passage of the implantable flowable composition through the perivascular space. Alternatively, a delivery device can be inserted into the extraluminal space to accomplish blunt dissection and the implantable flowable composition administered as the delivery device is retracted from the newly created planar administration area.

Vessel Visualization: The site of administration may be located with the assistance of a guidance system, for example an endoscopic guidance system, for example, intra-vascular ultrasound (IVUS). Intra-vascular ultrasound provides a 360° cross-sectional image of a blood vessel lumen, including surrounding structures and vessels.

The endoscopic guidance system may be angiography. A contrast agent may be added to the particulate cell suspension to permit imaging of the penetrating device and determination of the position of the penetrating device and the placement of the particulate cell suspension within a patient's body using, for example, contrast angiography.

Additional endoscopic guidance systems to locate the site of extraluminal administration, include, but are not limited to, color Doppler ultrasound, duplex ultrasound, other routine ultrasound, magnetic resonance angiography (MRA), magnetic resonance imaging (MRI), CT scanning, fluoroscopy to identify the location of a stent and/or other endoscopic guidance systems known in the field. Additionally, the site of administration may be located using tactile palpation.

The implantable flowable composition can be visualized within the extraluminal space following administration using, for example, angiography or IVUS. According to one embodiment, post-administration visualization is conducted to ensure the implantable flowable composition has been delivered to the extraluminal space rather than to the luminal space.

Dosage: Each deliverable administration of the flowable composition may contain about 1 x 10⁶ cells in a volume of about 50-60 mg of implantable particles. It is contemplated that either a single administration or multiple administrations of the flowable composition can be delivered to a single treatment site. It is also contemplated that multiple administrations of the flowable composition can be delivered to a single patient. Variations in multiple administration include administering multiple administrations to a single treatment site, administering single or multiple administrations to a variety of treatment sites, and/or providing administrations during a single treatment event or over an extended course of treatment.

Each administration of the flowable composition will contain a void volume, a portion of the allocated administration volume that remains in the delivery device following administration. The void volume may range from about 1% to about 50% of the volume of flowable composition loaded into the delivery device. Taking the void volume into account, a greater number of cells and particles are loaded into a delivery device than are intended for actual administration to a patient. Of course, the void volume and the attendant adjustment in the volume of flowable composition loaded into the delivery device will depend on the chosen delivery device.

Each deliverable administration of the flowable composition may be packaged as a single administration. When multiple doses are required for administration to a single treatment site, the desired dosage amount can be loaded into a single delivery device and administered during a single administration. However, when multiple doses are required for administration to multiple treatment sites within a single patient, it is preferable to use multiple aliquots of a fixed amount of deliverable product over multiple divided administrations of a larger dosage. Advantages of fixed administration aliquots include reducing dosage inaccuracies introduced by separating a larger dosage into multiple smaller dosages, reducing dosage inaccuracies introduced by measurement limitations inherent in the delivery device and reducing the dosage variability introduced by the excess volume of transport media required for a larger aliquot of the composition. Additionally, dividing a larger dosage into smaller aliquots requires unnecessary manipulation of the composition, including breaking up adjacent particles, breaking up confluent cell monolayers and causing other injuries to the cells.

Multiple administrations of the implantable flowable composition can be provided over an extended course of treatment. An initial dose of implantable flowable composition may be administered at the time of primary treatment or vascular intervention, followed by subsequent minimally invasive administrations once every 1-3 months, or as needed as determined by a clinician.

Backflow: A preclinical study was performed using a single porcine test subject undergoing endovascular administration of Gelfoam particles. A suspension of hydrated Gelfoam particles, mixed with contrast agent, was loaded into a catheter-based delivery mechanism, and the catheter was inserted into the vasculature of the test subject. The catheter was directed to the treatment site, the needle was inserted through the vessel wall, into the perivascular space, and the suspension was injected into the perivascular space. The injection procedure and follow-up were visualized with contrast angiography.

No backflow of the suspension into the vessel lumen was evident from the contrast angiography, either at the time of injection or following administration and removal of the needle. Furthermore, later histological evaluation of the treated tissue sections, stained with Verhoeff's elastin stain, showed no evidence of the suspension escaping from the adventitia into either the vessel lumen or into the surrounding tissues.

It is contemplated that a therapeutic amount of the implantable flowable composition, about 0.1 ml to about 2 ml, can be injected into the perivascular space at a single injection site before the pressure from the perivascular space is sufficient to result in backflow of the implantable flowable composition into the vessel lumen.

### Example 1: Animal Vascular Intervention Study.

This example provides experimental protocols for testing and using a preferred embodiment of the present invention to reduce the incidence of clinical sequelae associated with vascular intervention in animal test subjects. Using standard surgical procedures, an injury to the interior lumen surface is induced by percutaneous balloon angioplasty and placement of a stent performed on the femoral arteries. The implantable flowable composition of the present invention is then disposed in the perivascular space adjacent the site of angioplasty and stent treated vessel; the details of one exemplary procedure are set forth below. As described earlier, the placement and formulation of implantable flowable composition can be varied.

Specifically, the study includes 26 porcine test subjects undergoing percutaneous balloon angioplasty and stent implantation. Conventional percutaneous balloon angioplasty and stent implantation procedures will be performed according to standard operative techniques. Implantable flowable composition will be applied to the site of balloon inflation and stent placement and surrounds as described below after the angioplasty and stenting is completed and flow through the treated vessel is established.

Surgical Procedure: For each test subject undergoing percutaneous balloon angioplasty and stent implantation, the subject will be intubated and connected to a cardiac monitor device in the supine position. Right carotid arterial access with a 7 French sheath will be obtained via cutdown, and an 5.0 mm-diameter angioplasty balloon (Guidant Corp., Indianapolis, IN) advanced to the femoral artery under fluoroscopic guidance. Angiography will be performed and recorded by cineradiography. The right and left femoral arteries will be injured by 30-second balloon inflations between 8 to 10 atmospheres (3 inflations per side, in overlapping segments). Megalink biliary stents (Guidant, 6.0-8.0 mm x 18 mm) will be advanced to the femoral arteries under fluoroscopic guidance and placed at the site of angioplasty.

After angioplasty and placement of the stent, flowable composition comprising endothelial cells and particulate matrix, particulate matrix alone, or nothing will be delivered to the perivascular aspect of the left and right femoral arteries by a needle injection catheter. The needle injection catheter will be guided to the site of administration using, for example, angiography or intravascular ultrasound to identify the location of the stent. The implantable flowable composition will be administered at a location proximal to the stent, for example, a location about 1-20 mm proximal to the proximal end of the stent, at a location distal to the stent, for example, a location about 1-20 mm distal to the distal end of the stent, and at a site along the length of the stent. Each injection location will receive about 0.1-1.0 ml of implantable flowable composition containing approximately 40-70 mg particles at a density of about 0.8 - 2.5 x 10⁴ cells/mg. All test subjects will receive intra-operative heparin and administered daily aspirin following surgery.

Ten of the test subjects will receive implantable flowable composition on the day of surgery. Ten test subjects will receive control particulate matrix alone on the day of surgery. An additional 6 test subjects will receive a stent, but will not receive either type of implant. These 6 test subjects will be used for comparison to standard of care. The total cell load based on body weight will be approximately 1-8 x 10⁴ cells per kg.

Following completion of the angioplasty procedure, placement of the stent, and injection of the implantable flowable composition in the adjacent perivascular space, a C-arm fluoroscope will be placed over the neck of the subject so that the treated vessel will be visualized. Under continuous fluoroscopy, 10-15 cc's of iodinated contrast (Renograffin, full strength) will be injected. The cine angiography will be recorded and stored for comparison to the pre-sacrifice angiogram. Final angiography will be performed to evaluate vessel patency and the condition of the implantable particulate material injection sites.

Heparin will be administered prior to angioplasty as a 100 U/kg bolus injection plus a 35 U/kg/hr continuous infusion and maintained until the end of surgery. Additional bolus doses (100U/kg) will be administered, as necessary to maintain ACTs ≥200 seconds.

Vessel Patency: The patency of a treated vessel will be confirmed by access flow measurements using color-flow Doppler ultrasound immediately after surgery, 3-7 days post surgery and once per week thereafter. Treated vessels will be monitored closely for blood flow. Flow through the vessel must be detected up to and including day 7 post-surgery for the test subject to be placed on study. If flow is not detected through a vessel before or on day 7, the test subject will be removed from the study and every attempt made to replace the test subject so that the original number of subjects/group is maintained.

Pathology Procedures: Half of the animal test subjects (5 treated; 5 control; 3 subjects with no implant) will be euthanized 3-5 days following surgery. The remaining animal test subjects (5 treated; 5 control; 3 subjects with no implant) will be euthanized one month following surgery.

Animal test subjects will be anesthetized using sodium pentobarbital (65mg/kg, IV)). The treated vessel will be exposed and digital photography of the treated vessel and the surrounding tissue and vasculature performed. A C-arm fluoroscope will then be placed over the neck of the animal so that the treated vessel can be visualized. Under continuous fluoroscopy, 10-15 cc's of iodinated contrast (Renograffin, full strength) will be injected. The cine angiography will be recorded at 0° and 90° angles to the treated vessel. Vessel patency and degree of stenosis will be determined by blinded read of the necropsy angiograms in paired comparison with post-placement angiograms. Angiograms were graded on a scale of 0-5 depending upon the degree of stenosis observed in the angiogram. The grading scheme employed was as follows: 0 = 0% stenosis, 1 = 20% stenosis, 2 = 40% stenosis, 3 = 60% stenosis, 4 = 80% stenosis and 5 = 100% stenosis. It is anticipated that the vessels treated with the implantable flowable composition of the present invention will exhibit a decreased stenosis compared to control subjects upon examination of the angiograms.

Histology: Half of the animal test subjects (5 treated; 5 control; 3 subjects with no implant) will be euthanized 3 days following surgery. The remaining animal test subjects (5 treated; 5 control; 3 subjects with no implant) will be euthanized one month following surgery.

A limited necropsy, defined as the macroscopic examination of the administration site and surrounding tissue including draining lymph nodes will be performed on all test subjects. Tissue from major organs, including brain, lungs, kidneys, liver, heart and spleen, will be collected and saved for all test subjects euthanized at one month following surgery. The organs are to be analyzed only if unusual findings arise from macroscopic examination of the external surface of the body or from the microscopic examination of administration sites and surrounding tissue.

All treated vessels and surrounding tissues will be trimmed, fixed in 10% formalin (or equivalent) and embedded in glycolmethacrylate (or equivalent). Using approximately 3µm-thick sections cut with a C-profile stainless steel knife (or equivalent), sections will be prepared from each of three segments of the treated vessel: proximal to the injectable material; at the site of injectable material; and distal to the injectable material. These sections shall be mounted on gelatin-coated (or equivalent) glass slides and stained with hematoxylin and eosin or Verhoeff's elastin stain. The stained slides will be examined and scored for perivascular and luminal inflammation (acute and chronic), vascular degeneration, thrombi and fibrosis and for the presence of smooth muscle cells and endothelial cells.
Additional sections of tissue derived from the one-month test subjects will be stained with Verhoeff's elastin and examined and scored for the size of the vessel, and the extent of vessel injury, intimal hyperplasia and restenosis. Additional sections may also be stained with specific endothelial and smooth muscle cell markers, including but not limited to PECAM-1 and α-SMC actin. The residual lumen will also be examined, reflecting the change in vessel geometry after injury and repair. The Verhoeff's stained sections will also be subjected to Morphometric analysis using computerized digital planimetry with a video microscope and customized software.

Perivascular and luminal inflammation will be determined both acutely (3-5 day subjects) and chronically (1 month subjects). Acute inflammation is marked by granulocytes, primarily neutrophils, while chronic inflammation is marked by macrophages and lymphocytes. Additionally, sections may also be stained with the following specific markers: anti-CD45 to identify leukocytes, anti-CD3 to identify T cells, CD79a to identify B cells and MAC387 to identify monocytes/macrophages.

The stained slides will be examined and scored for the presence of smooth muscle cells and endothelial cells. All sections of the treated vessel, including the intima/pseudointima, the inner portion of the media near the lumen, the outer (adventitial site) portion of the media near the adventitia, and the adventitia will be evaluated and scored. The size of each tissue compartment, for example, the intima, the media and the adventitia, will be measured in microns. Each section will be evaluated for the presence and/or extent of each of the following criteria. Indicia of inflammation will be evaluated, including but not limited to, the presence and extent of neutrophils, lymphocytes, macrophages, eosinophils, giant cells and plasma cells. Tissue sections will be evaluated for the presence of fibroblasts, neovascularization, calcification, hemorrhage, congestion, fibrin, graft fibrosis and graft infiltration. Tissue sections additionally will be evaluated for indicia of degeneration, including but not limited to the degeneration, elastin loss and/or the absence of the tissue portion, smooth muscle myofiber vacuolation and/or calcification of the tissue. Tissue sections also will be evaluated for endothelial cell proliferation, subintimal cell proliferation, including but not limited to neovascularization and the presence of smooth muscle myofiber, fibroblasts and fibrosis. Each of the measured tissue sections also will be evaluated for tissue necrosis and the presence of foreign material. Scores will be assigned for each variable on a scale of 0 through 4 (0 = no significant changes; 1 = minimal; 2 = mild; 3 = moderate; and 4 = severe).

Additional sections of tissue from the 1-month animal test subjects only will be mounted on glass slides and stained (Verhoeff's elastin) for morphometric analysis. Measurements of the lumenal, medial, intimal and total vessel volume will be taken using computerized digital planimetry with a video microscope and customized software for each section. The percent stenosis will be determined for each section. One method of quantifying intimal hyperplasia is by dividing the intima area by the area of the intima and lumen [(intima, mm²) / (intima + lumen, mm²)].

Additional sections will also be obtained, at the discretion of the pathologist, if upon gross examination of the vessel(s) any focal lesions, thinning of the vessel wall(s) or dilation are observed outside of the sites described above. All stained slides will be examined and scored in blinded fashion by a board certified Veterinary Pathologist.

### Expected Results for Animal Vascular Intervention Subjects

It is expected that subjects treated with the flowable composition of the present invention as described above will display one or more indicia of reduced incidence of clinical sequelae associated with vascular intervention, including but not limited to decreased occlusive thrombosis, increased patency rates, decreased stenosis, decreased intimal hyperplasia, and decreased acute and chronic luminal and/or perivascular inflammation.

Another indicia of a successfully treated vessel is adequate lumen diameter. It is expected that the injectable material of the present invention will permit maintenance of adequate lumen diameter by reducing vessel stenosis and thereby permitting unimpeded blood flow at normal or near normal rates. Lumen diameter and percent stenosis will be monitored using angiography of the treated vessel at the day of treatment and just prior to 30-day sacrifice. Narrowing of the lumen post-surgery will be correlated with blood flow rates using standard Doppler ultrasound protocols. It is expected that the present invention will prevent or delay narrowing that impedes blood flow below a normal or near normal rate.

A further indicia of a functioning stented vessel is the absence of edge effects. It is expected that the injectable material of the present invention will reduce the incidence and/or extent of occlusive thrombosis or stenosis of the treated vessel in the portion of the vessel distal and proximal to the stent, often referred to as edge effects. Edge effects, or candy wrapper effects, are areas of stenosis that develop at the stent articulations and edges following stent placement.
Characterized by early neointimal tissue proliferation and later stenosis, edge effects can lead to occlusive thrombosis. Edge effects will be monitored using angiography of the treated vessel at the day of treatment and just prior to 30-day sacrifice. It is expected that the present invention will prevent or delay thrombosis and vessel narrowing associated with edge effects of stented vessels, as described herein. The presence or absence of edge effects will also be determined histologically by obtaining far-proximal and far-distal sections, approximately 2-3 mm upstream or downstream of the stent edges in either direction. To evaluate the presence of edge effects, far-proximal and far-distal sections will be scored for injury, inflammation, neointimal formation and thrombus.

As a group, the treated subjects are expected to show at least incremental differences in at least one of these aforementioned indicia of functionality as compared to controls.

### Example 2: Human Vascular Intervention Study.

This example provides experimental protocols for testing and using a flowable composition comprising engrafted vascular endothelial cells and a biocompatible matrix in particulate form to reduce the incidence of clinical sequelae associated with vascular intervention in human clinical test subjects. Using standard surgical procedures, a physician-ordered percutaneous balloon angioplasty and stenting is performed to alleviate a clinical condition. Implantable flowable composition is then disposed in the perivascular space at, adjacent or in the vicinity of the site of angioplasty and stenting; the details of one exemplary procedure are set forth below. As described earlier, the placement and formulation of the implantable flowable composition can be varied by the skilled practitioner in a routine manner.

Specifically, the study includes human test subjects undergoing percutaneous balloon angioplasty and stenting in a peripheral limb. Conventional percutaneous balloon angioplasty and stenting procedures will be performed according to standard operative techniques. The implantable flowable composition of the present invention will be applied to the site of balloon inflation and surrounds as described above after the angioplasty and stenting is completed and flow through the treated vessel is established. The total cell load based on body weight will be approximately 1.0 x10⁴ cells per kg to approximately 8.0 x10⁴ cells per kg.

Clinical follow-ups will be performed at 5 days, 2 weeks and at 1, 3 and 6 months. Blood flow measurements using color-flow Doppler ultrasound will be required at day 5 to establish a baseline level, followed at 2 weeks, 1 month, 3 months and 6 months post-surgery. Test subjects that exhibit an absolute flow of less than 350 mL/min, or greater than 25% reduction in flow from the previous measurement, or greater than 50% area stenosis (as measured by Doppler ultrasound) will be referred for angiography. Remedial clinical intervention such as angioplasty will be permitted for stenotic lesions of greater than 50% determined by angiography.

Contrast angiography of the treated vessel and surrounding tissue and vasculature will be performed at baseline and at 3 months. Lumen diameter will be calculated for each region and peak systolic velocity will be measured.

### Expected Results for Human Vascular Intervention Study

It is expected that subjects treated with the implantable flowable composition of the present invention as described above will display one or more indicia of reduced incidence of clinical sequelae associated with vascular intervention, including but not limited to occlusive thrombosis, restenosis, intimal hyperplasia, and acute and chronic inflammation.

An indicia of a functioning blood vessel is adequate lumen diameter. It is expected that the present invention will permit maintenance of adequate lumen diameter thereby permitting unimpeded blood flow at rates sufficient to maintain normal or near normal peripheral circulation. Lumen diameter will be monitored using angiography of the treated vessel beginning at baseline (approximately 5 days post-treatment) and thereafter at least 3 months post surgery. Narrowing of the lumen post-surgery will be correlated with blood flow rates using standard Doppler ultrasound protocols. It is expected that the implantable flowable composition of the present invention, when used as described herein, will prevent or delay narrowing that impedes blood flow below a rate suitable for peripheral circulation as described herein. It is further expected that treatment with the implantable flowable composition of the present invention will result in blood flow rates permitting clinically-acceptable circulation, or approximating normal rates. Flow into and out of a treated portion of a blood vessel will be comparable. Comparable means substantially similar for clinical purposes. For example, blood flow rates of about 150-500 mL/min, preferably about 300-500 mL/min, and more preferably about 350-400 mL/min.

As a group, the treated subjects are expected to show at least incremental differences in at least one of these aforementioned indicia of functionality as compared to controls.

### Example 3: Animal Pelvic Readhesion Study

This example provides experimental protocols for testing and using a flowable composition comprising a biocompatible particulate matrix and engrafted endothelial cells or endothelial-like cells to reduce the incidence of adhesions in the pelvis and its surrounds in animal test subjects. An experimental rat model (a modified uterine horn model, J. Invest. Surg. 7:409-15 (1994)) will be utilized to study the treatment of post-operative adhesions after tubal reconstructive surgery using the implantable flowable composition and methods of the present invention.

The uterine horn will be scratched on both sides and sutured together. After 14 days, during relaparotomy, the tight connection between the two sides of the sutured uterine horn will be cut. The implantable flowable composition of the present invention will be applied to one side of the uterine horn and surrounds as described above. The other side of the uterine horn will not receive the implantable flowable composition as a control. The presence or absence of adhesions will be monitored over time. It is expected that rats treated with the implantable flowable composition of the present invention will display a reduced incidence of adhesions in the pelvis and its surrounds.

### Example 4: Animal Fallopian Tube Occlusion Study

This example provides experimental protocols for testing and using a biocompatible particulate matrix and engrafted endothelial or endothelial-like cells to reduce the incidence of fallopian tube occlusion in animal test subjects. An experimental rabbit model (J. Vasc. Interv. Radiol. 13:399-404 (2002)) will be utilized to study the treatment of fallopian tube occlusion using the implantable flowable composition and methods of the present invention. Under fluoroscopic guidance, transvaginal catheterization of the right and left fallopian tube will be performed using a coaxial technique. With a metal guidewire protruding from the active electrode catheter, RF electrocoagulation will be performed. The implantable flowable composition of the present invention will be applied to one fallopian tube and surrounds as described above. The other fallopian tube will not receive the implantable flowable composition as a control. Tubal patency and histological changes will be evaluated over time. It is expected that rabbits treated with the implantable flowable composition of the present invention will display a reduced incidence of occlusion, stenosis and necroses in the fallopian tubes and their surrounds.

The present invention can also be used effectively to diminish the incidence of ectopic pregnancies and/or as an interventional therapy coincident with or following an ectopic pregnancy.

The invention may be embodied in other specific forms. The present embodiments are therefore to be considered illustrative and not restrictive, the scope of the invention being indicated by the appended claims rather than by the foregoing description.

## Claims

1. A flowable composition comprising a biocompatible matrix and endothelial cells or cells having an endothelial-like phenotype for use in the treatment of an ischemic tissue wherein said flowable composition is delivered by percutaneous injection to an extraluminal site at or adjacent or in the vicinity of the ischemic tissue in an amount effective to treat the ischemic tissue.

2. The flowable composition of claim 1 for use in the treatment of peripheral ischemia.

3. A flowable composition comprising a biocompatible matrix and cells for use in the treatment of an injured or diseased site on a non-luminal surface of a tubular anatomical structure wherein said flowable composition is delivered by percutaneous injection to an extraluminal surface at or adjacent or in the vicinity of the injured or diseased site in an amount effective to treat the injured or diseased site.

4. The composition of claim 3 wherein the injured or diseased site is within the adventitia, media or intima or on an exterior surface of a blood vessel.

5. The composition of claim 3 or claim 4 wherein the tubular anatomical structure is selected from: lacrimal ducts, the trachea, bronchi, bronchiole, nasal passages, sinuses, airways, eustachian tubes, the external auditory canal, oral cavities, the oesophagus, the stomach, the duodenum, the small intestine, the large intestine, biliary tracts, the ureter, the bladder, the urethra, the fallopian tubes, uterus, vagina and other passageways of the female reproductive tract and the vasdeferens and other passages of the male reproductive tract.

6. A flowable composition comprising a biocompatible matrix and endothelial cells or cells having an endothelial-like phenotype for use in the treatment of a vascular injury resulting from vascular intervention wherein said flowable composition is delivered by percutaneous injection to an extraluminal surface at or adjacent or in the vicinity of the site of vascular injury in an amount effective to treat the injured site.

7. The composition of claim 6 wherein the vascular injury results from a vascular anastomosis, for example an arteriovenous graft, an arteriovenous fistula or a peripheral graft.

8. The composition of claim 6 wherein the vascular injury results from an arterial bypass graft or a peripheral bypass graft.

9. The composition of claim 6 wherein the vascular injury results from an organ transplantation.

10. The composition of claim 6 wherein the vascular injury results from angioplasty, atherectomy, or vascular stenting.

11. The composition of any one of the preceding claims wherein the composition is delivered by a minimally invasive closed procedure,

12. The composition of any one of claims 3-5 wherein the cells are endothelial cells or cells having an endothelial-lilce phenotype.

13. The composition of any one of the preceding claims wherein said cells are a confluent population of cells, a near-confluent population of cells, a post-confluent population of cells or a population of cells having any one of the foregoing phenotypes.

14. The composition of any one of the preceding claims wherein the biocompatible matrix comprises particles or microcarriers.

15. The composition of any one of the preceding claims wherein the biocompatible matrix comprises gelatin, collagen, fibronectin, fibrin, laminin, an attachment peptide or a combination of any of the foregoing.

16. The composition of any one of the preceding claims wherein said effective amount of the flowable composition reduces smooth muscle cell proliferation, reduces occlusive thrombosis, reduces intimal hyperplasia, reduces stenosis or restenosis, reduces acute inflammation, reduces chronic inflammation, or reduces vasodilation or vasospasm at the injured or diseased site.

17. An injection-type delivery device comprising:
(a) a 22-26 guage internal diameter needle; and
(b) a flowable composition comprising a suspension of gelatin particles having a diameter of about 100 µm to about 500 µm, said particles having endothelial cells or cells having an endothelial-like phenotype adhered thereto;
said device being suitable for use in a method of treating an injured or diseased site by extraluminal or non-luminal deposition of said flowable composition at, adjacent or in the vicinity of said injured or diseased site in an amount effective to treat said injured or diseased site.

18. The device of claim 17 wherein the method of treating the injured or diseased site is as defined in any one of claims 1 to 11.

19. The device of claim 17 or claim 18 wherein said cells are as defined in claim 13.

## Patentansprüche

1. Fließfähige Zusammensetzung, die eine biokompatible Matrix und Endothelzellen oder Zellen mit einem Endothel-ähnlichen Phänotyp umfasst, zur Verwendung bei der Behandlung von ischämischem Gewebe, wobei die fließfähige Zusammensetzung mittels perkutaner Injektion in eine extra-luminale Stelle an oder neben oder in der Nähe des ischämischen Gewebes in einer Menge verabreicht wird, die wirksam ist, das ischämische Gewebe zu behandeln.

2. Fließfähige Zusammensetzungnach Anspruch 1 zur Verwendung bei der Behandlung von peripherer Ischämie.

3. Fließfähige Zusammensetzung, die eine biokompatible Matrix und Zellen umfasst, zur Verwendung bei der Behandlung einer verletzten oder erkrankten Stelle auf einer nicht-luminalenOberfläche einer tubulären anatomischen Struktur, wobei die fließfähige Zusammensetzung mittels perkutaner Injektion in eine extra-luminale Oberfläche an oder neben oder in der Nähe des verletzten oder erkrankten Gewebes in einer Menge verabreicht wird, die wirksam ist, das verletzte oder erkrankte Gewebe zu behandeln.

4. Zusammcnsctzungnach Anspruch 3, wobei sich das verletzte oder erkrankte Gewebe in der Adventitia, der Media oder der Intima oder auf einer äußeren Oberfläche eines Blutgefäßes befindet.

5. Zusammensetzungnach Anspruch 3 oder Anspruch 4, wobei die tubuläre anatomische Struktur ausgewählt ist unter: Tränenleiter, der Luftröhre, den Bronchien, den Bronchiolen, den Nasenleitungen, den Stirnhöhlen, den Ohrtrompeten, dem externen Höhrkanal, der Mundhöhle, dem Oesophagus, dem Magen, dem Duodenum, dem Dünndarm, dem Dickdarm, den Gallenleitern, dem Harnleiter, der Blase, der Harnröhre, den Eileitern, dem Uterus, der Vagina und anderen Gängen des weiblichen Fortpflanzungstrakts und den Samenleitern und anderen Gängen des männlichen Fortpflanzungstraktes

6. Fließfähige Zusammensetzung, die eine biokompatible Matrix und Endothelzellen oder Zellen mit einem Endothel-ähnlichen Phänotyp umfasst, zur Verwendung bei der Behandlung einer vaskulären Verletzung, die auf eine vaskuläre Intervention zurückgeht, wobei die fließfähige Zusammensetzung mittels perkutaner Injektion in eine extraluminaleOberfläche an oder neben oder in der Nähe der Stelle der vaskulären Verletzung in einer Menge verabreicht wird, die wirksam ist, die vaskuläre Verletzung zu behandeln.

7. Zusammensetzungnach Anspruch 6, wobei die vaskulären Verletzung auf eine vaskuläre Anastomose, beispielsweise ein arteriovenöses Transplantat, eine arteriovenöse Fistel oder ein peripheres Transplantat zurückgeht.

8. Zusammensetzungnach Anspruch 6, worin die vaskuläre Verletzung auf ein arterielles Bypass-Transplantat oder ein peripheres Bypass-Transplantat zurückgeht.

9. Zusammensetzungnach Anspruch 6, wobei die vaskuläre Verletzung auf eine Organ-Transplantation zurückgeht.

10. Zusammensetzungnach Anspruch 6, wobei die vaskuläre Verletzung auf Angioplastie, Atherektomie oder eine vaskuläre Stent-Implantation zurückgeht.

11. Zusammensetzungnach einem der vorhergehenden Ansprüche, wobei die Zusammensetzung mittels geschlossenem, minimal-invasivem Verfahren verabreicht wird.

12. Zusammensetzungnach einem der Ansprüche 3 - 5, wobei die ZellenEndothelzellen oder Zellen mit einem Endothel-ähnlichen Phänotyp sind.

13. Zusammensetzungnach einem der vorstehenden Ansprüche, wobei die Zellen eine konfluente Population von Zellen, eine fast-konfluente Population von Zellen, eine postkonfluente Population von Zellen, oder eine Population von Zellen mit einem der vorstehenden Phänotypen sind.

14. Zusammensetzungnach einem der vorstehenden Ansprüche, wobei die biokompatible Matrix Teilchen oder Mikroträger enthält.

15. Zusammensetzungnach einem der vorstehenden Ansprüche, wobei die biokompatible Matrix Gelatine, Kollagen, Fibronektin, Fibrin, Laminin, ein Anheftungs-Peptid, oder eine Kombination der vorstehenden umfasst.

16. Zusammensetzungnach einem der vorstehenden Ansprüche, wobei die wirksame Menge der fließfähigen Zusammensetzung die Proliferation glatter Muskelzellen reduziert, okklusive Thrombose reduziert, intimale Hyperplasie reduziert, Stenose oder Restenose reduziert, akute Entzündung reduziert, chronische Entzündung reduziert, oder Vasodilatation oder Vasospasmus an der verletzten oder erkrankten Stelle reduziert.

17. Verabreichungs-Einrichtung vom Injektionstyp, welcher umfasst:
(a) eine Nadel mit 22-26 Innendurchmesser-Maß; und
(b) eine fließfähige Zusammensetzung, welche eine Suspension von Gelatinekapseln mit einem Durchmesser von etwa 100 µm bis etwa 500 µm aufweist, worin die Teilchen daran angeheftete Endothelzellen oder Zellen mit einem Endothel-ähnlichen Phänotyp umfassen:
worin die Einrichtung zur Verwendung in einem Verfahren zur Behandlung einer verletzten oder erkrankten Stelle mittels extra-luminaler oder nicht-luminaler Ablagerung der fließfähigen Zusammensetzung an, neben oder in der Nähe der verletzten oder erkrankten Stelle in einer Menge geeignet ist, die wirksam ist, die verletzte oder erkrankte Stelle zu behandeln.

18. Einrichtung nach Anspruch 17, worin das Verfahren zur Behandlung der verletzten oder erkrankten Stelle wie in einem der Ansprüche 1 bis 11 ist.

19. Einrichtung nach Anspruch 17 oder Anspruch 18, worin die Zellen wie in Anspruch 13 definiert sind.

## Revendications

1. Composition fluide comprenant une matrice biocompatible et des cellules endothéliales ou des cellules ayant un phénotype de type endothélial pour une utilisation dans le traitement d'un tissu ischémique, ladite composition fluide étant administrée par injection percutanée à un site extraluminal au niveau du ou adjacent au ou à proximité du tissu ischémique dans une quantité efficace pour traiter le tissu ischémique.

2. Composition fluide selon la revendication 1, pour une utilisation dans le traitement d'une ischémie périphérique.

3. Composition fluide comprenant une matrice biocompatible et des cellules pour une utilisation dans le traitement d'un site lésé ou malade sur une surface non luminale d'une structure anatomique tubulaire, ladite composition fluide étant administrée par injection percutanée à une surface extraluminale au niveau du ou adjacent au ou à proximité du site lésé ou malade dans une quantité efficace pour traiter le site lésé ou malade.

4. Composition selon la revendication 3, où le site lésé ou malade se situe au sein de l'adventice, la media ou l'intima ou sur une surface extérieure d'un vaisseau sanguin.

5. Composition selon la revendication 3 ou la revendication 4, où la structure anatomique tubulaire est choisie parmi : les canaux lacrymaux, la trachée, les bronches, les bronchioles, les voies nasales, les sinus, les voies respiratoires, les trompes d'Eustache, le conduit auditif externe, les cavités orales, l'oesophage, l'estomac, le duodénum, l'intestin grêle, le gros intestin, les canaux biliaires, l'uretère, la vessie, l'urètre, les trompes de Fallope, l'utérus, le vagin et d'autres voies du système reproducteur de la femme et les canaux déférents et d'autres voies du système reproducteur de l'homme.

6. Composition fluide comprenant une matrice biocompatible et des cellules endothéliales ou des cellules ayant un phénotype de type endothélial pour une utilisation dans le traitement d'une lésion vasculaire résultant d'une intervention vasculaire où ladite composition fluide est administrée par injection percutanée à une surface extraluminale au niveau du ou adjacent au ou à proximité du site de la lésion vasculaire dans une quantité efficace pour traiter le site lésé.

7. Composition selon la revendication 6, où la lésion vasculaire résulte d'une anastomose vasculaire, par exemple, une greffe artérioveineuse, une fistule artéricveineuse ou une greffe périphérique.

8. Composition selon la revendication 6, où la lésion vasculaire résulte d'un pontage artériel ou d'un pontage périphérique.

9. Composition selon la revendication 6, où la lésion vasculaire résulte d'une transplantation d'organe.

10. Composition selon la revendication 6, où la lésion vasculaire résulte d'une angicplastie, d'une athérectomie, ou de la pose d'une endoprothèse vasculaire.

11. Composition selon l'une quelconque des revendications précédentes, la composition étant administrée par une procédure fermée invasive de façon minimale.

12. Composition selon l'une quelconque des revendications 3 à 5, où les cellules sont des cellules endothéliales ou des cellules ayant un phénotype de type endothélial.

13. Composition selon l'une quelconque des revendications précédentes, où lesdites cellules sont une population confluente de cellules, une population presque confluente de cellules, une population post-confluente de cellules ou une population de cellules ayant l'un quelconque des phénotypes précédents.

14. Composition selon l'une quelconque des revendications précédentes, dans laquelle la matrice biocompatible comprend des particules ou des microsupports.

15. Composition selon l'une quelconque des revendications précédentes, dans laquelle la matrice biocompatible comprend de la gélatine, du collagène, de la fibronectine, de la fibrine, de la laminine, un peptide de fixation ou une combinaison de n'importe lesquels des précédents.

16. Composition selon l'une quelconque des revendications précédentes, où ladite quantité efficace de la composition fluide réduit la prolifération des cellules des muscles lisses, réduit la thrombose occlusive, réduit l'hyperplasie intimale, réduit la sténose ou la resténose, réduit l'inflammation aiguë, réduit l'inflammation chronique, ou réduit la vasodilatation ou le vasospasme au niveau du site lésé ou malade.

17. Dispositif d'administration de type injection comprenant :
(a) une aiguille de diamètre interne de 22 à 26 gauge ; et
(b) une composition fluide comprenant une suspension de particules de gélatine ayant un diamètre d'environ 100 µm à environ 500 µm, lesdites particules ayant des cellules endothéliales ou des cellules ayant un phénotype de type endothélial y adhérant ;
ledit dispositif étant approprié pour une utilisation dans un procédé de traitement d'un site lésé ou malade par dépôt extraluminal ou ncn luminal de ladite composition fluide au niveau dudit, adjacent au dit ou à proximité dudit site lésé ou malade dans une quantité efficace pour traiter ledit site lésé ou maladie.

18. Dispositif selon la revendication 17, où le procédé de traitement du site lésé ou malade est tel que défini dans l'une quelconque des revendications 1 à 11.

19. Dispositif selon la revendication 17 ou la revendication 18, dans lequel lesdites cellules sont telles que définies dans la revendications 13.
